# EUROPEAN PATENT SPECIFICATION

(11) **EP 3 911 249 B1**
(45) Date of publication and mention of the grant of the patent: **11.03.2026**
(21) Application number: 20704131.0
(22) Date of filing: 09.01.2020
(51) Int. Cl.: A61B 17/04

(54) **SUTURE CUTTING DEVICES**
NAHTSCHNEIDEVORRICHTUNGEN
DISPOSITIFS DE COUPURE DE SUTURE

(30) Priority: 14.01.2019 US 201962792053 P
(43) Date of publication of application: 24.11.2021
(73) Proprietor: Ceterix Orthopaedics, Inc., Fremont CA 94555 (US)
(72) Inventor: PETER, Stephen J., Fremont, California 94555 (US); QUITUGUA, Victoria C., Fremont, California 94555 (US); TAGGARD, Joshua D., Fremont, California 94555 (US); BENDER, Christopher P., Fremont, California 94555 (US); HIROTSUKA, Mark Y., Fremont, California 94555 (US); HENDRICKSEN, Michael J., Fremont, California 94555 (US)
(74) Representative: Appleyard Lees IP LLP
(86) International application number: PCT/US2020/012876
(87) International publication number: WO 2020/150066

(56) References cited:
- WO-A1-2017/025971
- US-B2- 7 491 212
- US-B2- 9 247 935

## Description

### CROSS REFERENCE TO RELATED APPLICATION

This application claims priority to US Patent Application No. 62/792053, entitled "SUTURE CUTTING DEVICES", filed January 14, 2019. This application may be related to U.S. Provisional Patent Application No. 61/881,319, entitled "ARTHROSCOPIC KNOT PUSHER AND SUTURE CUTTER," filed on September 23, 2013; U.S. Patent No. 9,247,935, entitled "ARTHROSCOPIC KNOT PUSHER AND SUTURE CUTTER," issued on February 2, 2016; U.S. Patent No. 9,332,980, entitled "ARTHROSCOPIC KNOT PUSHER AND SUTURE CUTTER," issued on May 10, 2016; U.S. Patent Application No. 16/208,526, entitled "ARTHROSCOPIC KNOT PUSHER AND SUTURE CUTTER," filed on December 3, 2018; and U.S. Patent No. 10,143,464, entitled "ARTHROSCOPIC KNOT PUSHER AND SUTURE CUTTER," issued on December 4, 2018.

### FIELD

Suture cutting devices, as well as methods of making and using suture cutting devices are described herein. In particular, suture cutting devices adapted for use in narrow, confined, and/or difficult to access regions of a body, such as a knee joint, are described herein.

### BACKGROUND

Suturing of tissue generally involves passing a suture, often by assistance of a needle, through a body tissue in a way that holds the body tissue together. Once the suture is stitched into the tissue, a surgical knot is typically used to join the ends of the suture to create a suture loop. The resultant knot is cinched to the repair site and tightened to approximate tissue and complete the repair. Then, the excess suture limbs are cut and removed from the tissue. Devices capable of suturing, knot pushing and suture cutting are typically used when performing these procedures.

Suturing of tissue can be particularly challenging in difficult to access body regions and regions that have limited clearance, such as regions partially surrounded or covered by bone. Such hard to reach areas include the joints of the body, including the knee (e.g., meniscus) and the shoulder. For many surgical procedures, it is necessary to make a large opening in the human body to expose the area requiring surgical repair. However, in many cases, accessing the tissue in this manner is undesirable, increasing recovery time, and exposing the patient to a greater risk of infection. Minimally invasive suture-related devices have been developed for accessing these hard to reach areas without the need to make large openings in the body. Such devices have specialized features for limiting the size of incisions needed to enter the body and performing suture-related functions within tight areas. Generally, these devices are small in size and have low profiles for entering the body and navigating between soft tissues and bone. In some cases, the small size constraints for these devices have an impact on the functioning of the devices. For instance, it can be difficult to provide the necessary forces to cut sutures in confined spaces without damaging closely surrounding tissues. Thus, there is a need for improved devices and methods for manipulating and cutting sutures, particularly on tissue in difficult to access regions of the body including the joints (shoulder, knee, etc.). US 9,247,935 B2 discloses an arthroscopic knot pusher and sutures cutter.

### SUMMARY OF THE DISCLOSURE

The invention is defined by claim 1.

The devices and methods described herein can be used to cut sutures during a medical procedure, such as during arthroscopic procedures. The suture cutting devices can have a low profile shape for accessing regions within the body with limited space like joints, such as the knee or shoulder. The suture cutting devices include a cutter (also referred to as a blade) that can have an end with a cutting edge. The cutter has a hollow (e.g., tubular) shape to accommodate a holding tube used to secure the suture. The cutter may be rotatable and/or axially translatable for cutting the suture. The rotation and axial translation of the cutter may be separately or simultaneously executed. The suture cutting devices may be configured to push the knot within the suture and secure the suture for cutting near the knot.

According to embodiments, a suture cutting device includes a handle and an elongate holding tube attached to the handle. The holding tube including a slot configured to allow a suture to pass into the holding tube. The suture cutting device includes an inner mandrel within the holding tube. The inner mandrel is configured to move relative to the holding tube to capture the suture in a channel between the inner mandrel and the holding tube. The suture cutting device including a tubular cutter around the holding tube. The tubular cutter is configured to move axially relative to the elongate holding tube. The cutter is configured to rotate relative to the holding tube such that a cutting edge of the cutter cuts the suture when captured within the channel. The suture cutting device includes an actuator configured to control rotation of the cutter for cutting the suture. The cutter is operationally coupled to a threaded collar that is configured to rotate and axially translate the cutter

In some cases, the cutter may be configured to simultaneously rotate and move axially. The cutter may be configured to separately rotate and move axially. The actuator may be configured to control rotation and axial movement of the cutter. The actuator may be configured to be activated in at least two modes, wherein activating the actuator in a first mode causes the cutter to rotate and activating the actuator in a second mode causes the cutter to move axially. Activating the actuator in the first mode can include rotating the actuator, and activating the actuator in the second mode can include translating the actuator. In some cases, the suture cutting device includes at least two actuators. For example, a first actuator can be configured to control rotation of the cutter and a second actuator can be configured to control axial movement of the cutter. The actuator may be configured to rotate the cutter in a first direction when the actuator is activated and rotate the cutter in a second direction when the actuator is released. The device can further include a cutter release configured to prevent rotation and/or axial motion of the cutter unless the cutter release is disengaged. The gear assembly can include a spring configured to bias the cutter axially in a distal direction relative to the handle. The gear assembly can include a second spring configured to bias the cutter axially in a proximal direction relative to the handle. The gear assembly can include at least a first gear operationally engaged with a second gear. For example, the first gear can be coupled to the cutter and the second gear can be coupled to the actuator. The first gear may share a rotational axis with the cutter. The actuator can include a slider that translates distally and/or proximally relative to the holding tube, wherein the second gear is configured to rotate in response to translation of the actuator. The actuator may be located anywhere on the suture cutting device. For example, the actuator can be on the on the handle. The actuator can include any type of actuator (also referred to as a control). For instance, the actuator can include one or more of a button, a translatable member, a turnable member or a rotatable member. The actuator can include one or more of a pushable button, a pullable button, a slider, a toggle, a knob, a lever, a trigger or a wheel. The suture cutting device can also include a lock coupled to the inner mandrel and configured to prevent axial movement of the inner mandrel until the lock is released. The lock can be configured to be released by the operation of the actuator. The inner mandrel can be axially movable relative to the holding tube. The holding tube can be configured to capture the suture between the notch of the inner mandrel and the holding tube when the inner mandrel is extended distally. The actuator may be configured to control axial motion of the inner mandrel. In some instances, the actuator is configured to be operated with a single finger. The cutter may be operationally coupled to a cam assembly that is configured to rotate and/or axially translate the cutter. In some cases, the rotation and/or axially translation of cutter is at least partially defined by a guide path. The guide path can include a channel in the cutter or connected to the cutter. The guide path can include a channel in or on the actuator. The inner mandrel may rotate with respect to the holding tube to capture the suture. The inner mandrel may rotate with respect to the holding tube to capture the suture. The inner mandrel may be configured to rotate without axially moving with respect to the holding tube. The inner mandrel may rotate without axially moving with respect to the holding tube to capture the suture. The inner mandrel rotates without axially moving distally with respect to the holding tube.

### BRIEF DESCRIPTION OF THE DRAWINGS

Novel features of embodiments described herein are set forth with particularity in the appended claims. A better understanding of the features and advantages of the embodiments may be obtained by reference to the following detailed description that sets forth illustrative embodiments and the accompanying drawings.
FIG. 1 shows a side perspective view of a suture cutting device.
FIGS. 2A-2E illustrate an exemplary operation of a suture cutter device.
FIG. 3 illustrates exemplary motions of a cutter as part of a suture cutting device.
FIGS. 4A-4C illustrate an exemplary operation of a gear assembly as part of a suture cutting device.
FIGS. 5A-5C illustrate an exemplary operation of a wheel actuator configured to rotate and translate a cutter.
FIGS. 6A-6D illustrate an exemplary operation of a wheel actuator configured to allow separate rotation and translation of a cutter.
FIGS. 7A-7C illustrate an exemplary operation of a variation of a wheel actuator configured to allow separate rotation and translation of a cutter.
FIGS. 8A and 8B illustrate another variation of a wheel actuator.
FIG. 9 illustrates further variation of a wheel actuator.
FIG. 10 illustrates an additional variation of a wheel actuator.
FIGS. 11A-11C illustrate an exemplary cam assembly as part of a suture cutting device.
FIG. 12 illustrates an exemplary threaded collar assembly as part of a suture cutting device.
FIGS. 13A-13D illustrate an exemplary operation of another threaded collar assembly.
FIGS. 14A-14F illustrate an exemplary operation of a further threaded collar assembly.
FIG. 15 illustrates an exemplary suture cutting device having an actuator that stores potential energy for rotating a cutter.
FIG. 16 illustrates a further exemplary gear assembly as part of a suture cutting device.
FIG. 17A-17E illustrate exemplary suture cutting devices where the cutter is an axle or is part of an axle of a wheel.
FIGS. 18A and 18B illustrate exemplary suture cutting devices including a twist knob.
FIGS. 19A-19F illustrate exemplary suture cutting devices having a guide path.
FIGS. 20A-20I illustrate an exemplary operation of another gear assembly as part of a suture cutting device.
FIGS. 21A-21E illustrate another exemplary gear assembly.
FIGS. 22A-22C illustrate a further exemplary gear assembly.
FIGS. 23A-23C illustrate an additional exemplary gear assembly.
FIGS. 24A-24E illustrate yet another exemplary gear assembly.
FIG. 25 illustrates a further exemplary gear assembly.
FIGS. 26A-26D illustrate another exemplary suture cutting device having a guide path.

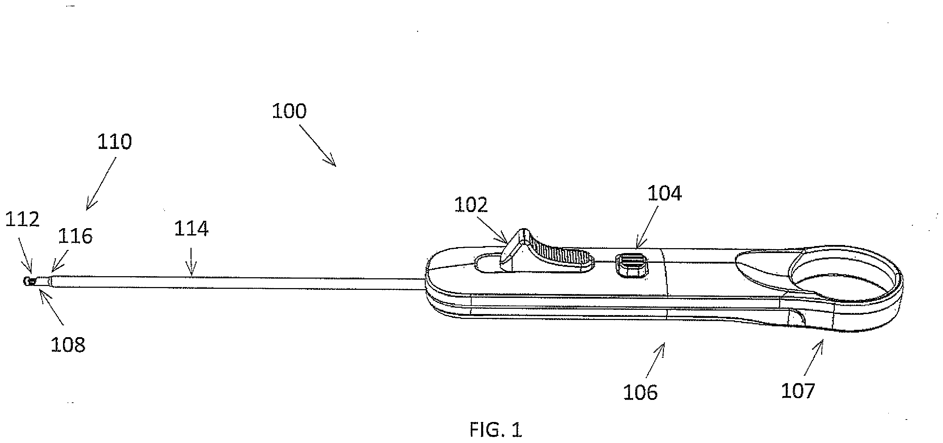

The features of any of the embodiments described herein can be combined any way. For example, any of the features described herein with respect to one or more of FIGS. 1-26D can be combined in any way.

### DETAILED DESCRIPTION

Devices and methods described herein can be used to cut sutures in a patient during a medical procedure. The devices can be shaped and sized to access confined regions of the body, such as joint areas (e.g., knees, shoulders, etc.). The devices include an elongate holding tube and a cutter that at least partially surrounds the holding tube and that can be configured to cut the suture after suturing of a body tissue. The cutter is configured to rotate with respect to the holding tube to cut the suture. Additionally, the cutter can be configured to advance distally with respect to the holding tube to cut the suture. In some embodiments, the devices can additionally be used to push a pre-tied knot within the suture to cinch the knot in a desired location, such as a repair site. The devices include an inner mandrel within the holding tube, which can cooperate with the holding tube to secure the suture during the cutting. The inner mandrel moves with the respect to the holding tube. For example, the inner mandrel may rotate with respect to the holding tube. In some cases, the inner mandrel rotates without axially moving (e.g., in a distal direction) with respect to the holding tube. The inner mandrel can include a lateral notch that accommodates the suture and holds the suture to an inner wall of the holding tube.

Any of the devices described herein may be dimensioned for use with surgical sutures placed in a joint, such as the knee, and may further be dimensioned for arthroscopic use. For example, the outer diameter of the inner mandrel (except the lateral notch region, e.g., in some variations a narrower region forming the laterally extending space, for holding the suture in conjunction with the inner wall of the outer holding tube), may be between about 0.5 and 4 millimeters (mm) (e.g., about 2 mm). The inner diameter of the cutter may be between about 1 mm and about 5 mm (e.g., 2.75 mm). The elongated length of the holding tube may be between about 6 and about 20 centimeters (cm), e.g., between about 10 cm and about 11 cm.

FIG. 1 illustrates an exemplary suture cutting device 100 as described herein. In this example, the device 100 includes an elongate holding tube 108 attached to a handle 106. The suture is loaded into a distal end 110 of the holding tube 108. The holding tube 108 can include a slot 112 (lateral opening) that is configured to allow the suture to pass therethrough. An inner mandrel within the holding tube can be configured to facilitate securing of the suture at the distal end 110 of the holding tube 108. A cutter 114 having a cutting edge 116 can be positioned around the holding tube 108. The cutter 114 can have any shape. For example, the cutter 114 can have a tubular shape, or partially tubular shape. A tubular or partially tubular shaped cutter 114 can have a hollow (e.g., central hollow) that can be configured to accommodate the holding tube 108 therein. In other cases, the cutter is not positioned around the holding tube 108. In some embodiments, the cutter does not include a hollow (e.g., solid cylindrical shape). A cross section of the cutter 114 can have any shape. For example, the cutter 114 can have a circular, elliptical, oval, polygonal (e.g., square, rectangular, hexagonal, pentagonal) or irregular cross section. In some embodiments, the cutter 114 is configured to rotate relative to the holding tube 108 such that the cutting edge 116 cuts the suture. In some embodiments, the cutter 114 is configured to move axially relative to the holding tube 108. For example, the cutter 114 may be configured to advance distally to cut the suture. In some cases, the cutter 114 is configured to simultaneously rotate and advance distally relative to the holding tube 108. In some embodiments, the device 100 is configured to push a surgical knot within the suture, for example, prior to cutting the suture.

The suture cutting device 100 can include an actuator 102 for controlling the cutting of the suture. The actuator 102 may be referred to herein as a control. The actuator 102 can be a switch. In some cases, the actuator 102 is on the handle 106. In some cases, the actuator 102 is on a component located between the handle 106 and the distal end 110. In some cases, the actuator 102 is on a component located separate from the handle 106, such as a remote control. The actuator 102 can be configured to move the inner mandrel for suture loading and/or to actuate the cutter 114 to cut the suture. Although the actuator 102 in FIG. 1 is a slider, the suture cutting devices described herein can be of any type actuator. For example, the actuator can include a mechanical switch, electrical switch, or both. In some embodiments, the actuator includes a button (e.g., pushable or pullable), a translatable member (e.g., slider or toggle), a turnable or rotatable member (e.g., knob, lever, trigger or wheel). In some embodiments, the suture cutting device includes more than one actuator (e.g., two, three, four or five). The suture cutting device 100 may optionally include a cutter release 104 that is configured to prevent rotation and/or axial motion of the cutter 114 unless the cutter release 104 is disengaged. The cutter release 104 may be used as a safety feature. In some embodiments, the cutter release 104 is configured to prevent distal movement of the actuator 102 and/or cutter 114 until the cutter release 104 is activated (e.g., pressed). In some cases, the proximal end of the handle 106 includes a thumb ring 107 that may be used to hold and manipulate the device 100. The handle 106 can advantageously be held still and/or kept in position throughout the surgery without requiring rotation or repositioning of the surgeon's hand relative to the handle 106. The same hand can be used to hold the device 100 and activate the actuator 102 and/or cutter release 104.

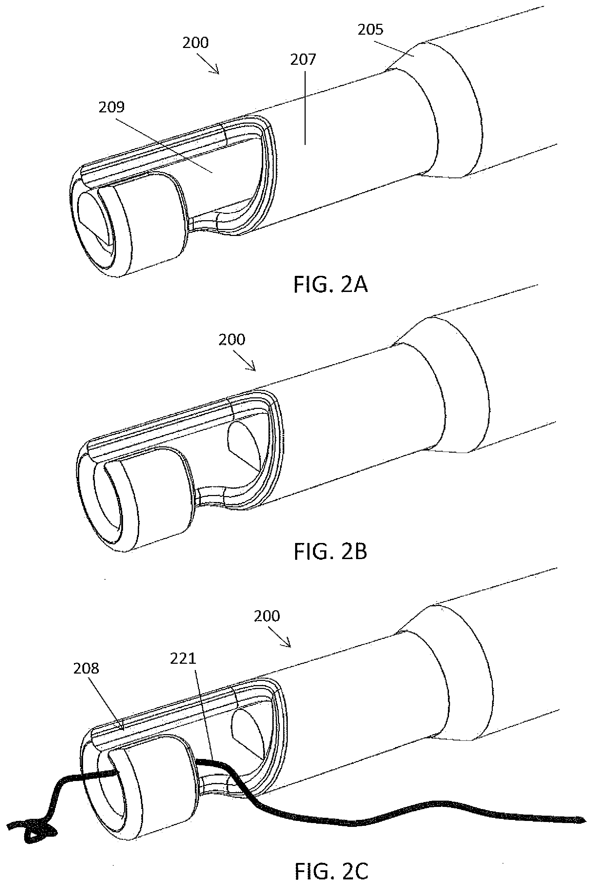

FIGS. 2A-2E illustrate an exemplary operation of a cutting device 200. In this example, the distal end of the device is shown, including the holding tube 207 and the inner mandrel 209. In FIG. 2A the inner mandrel 209 is shown at rest, biased distally by a compression spring, e.g., so that the distal end of the inner mandrel 209 is flush (or approximately flush) with the distal end of the holding tube 207. The inner mandrel 209 may be locked by a lock (e.g., lock out tab, button or switch). In some embodiments, the lock is configured to be locked and/or released by the operation of the actuator. To load a suture, the inner mandrel 209 may first be axially slid proximally, as shown in FIG. 2B. In this example, the actuator on the handle may first be moved to first disengage the lock, and after it has disengaged, to slide the inner mandrel 209 proximally. Alternatively, a separate actuator may be used to disengage the lock, for example, a button that deflects the lock so that it does not secure to the inner mandrel 209.

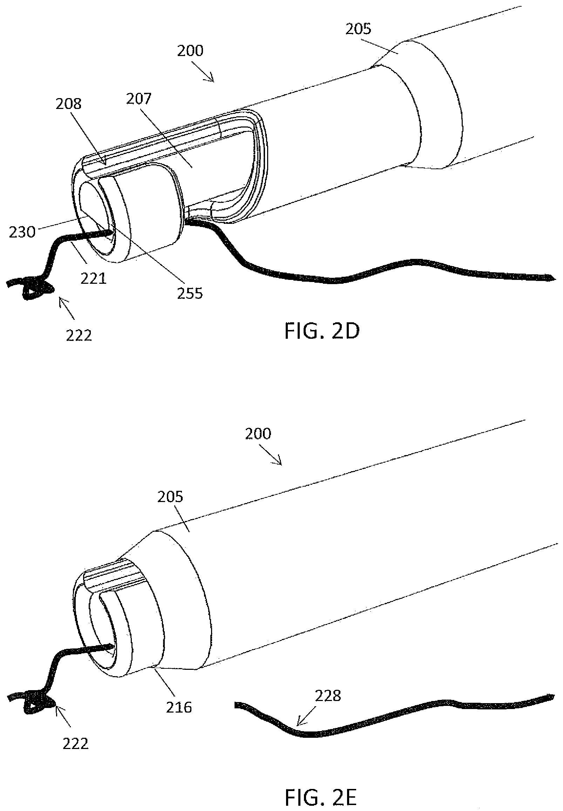

**As** shown in FIG. 2C, the suture 221 may then be loaded through a slot 208 of the holding tube 207 and into the inner chamber of the holding tube 207. In some cases, the opening of the slot 208 extends proximally to form a "J" or "L" shaped slot, which can facilitate the capturing of the suture 221. At FIG. 2D, the inner mandrel 209 may be released (e.g., by releasing the actuator) so that it moves axially (returns) to a distal position, with the suture 221 trapped in a channel 255 between the inner mandrel 209 and an inner wall of the holding tube 207. In some cases, the device 200 may also be used to push a knot 222 (e.g., pre-tied slideable knot) within the suture 221 distally to cinch the knot 222. The inner mandrel 207 may have a notch 230 along a side of the inner mandrel 207 to facilitate the capturing of the suture 221. In this example, the notch 230 is a flat region on the mandrel which creates enough space for the suture 221 within the channel 255, but not enough so that the knot 222 can pass through the channel 255. In some cases, the inner mandrel 207 is rotatable (e.g., by 180 degrees) to manipulate the location of the suture 221 within the channel 255 (e.g., so that the suture 221 reside adjacent to the slot 208. Once the suture 221 is positioned, the cutter 205 may be actuated (e.g., by releasing the lock and operating the actuator on the handle) such that a cutting edge 216 of the cutter 205 cuts the suture 221, as illustrated in FIG. 2E.

It should be noted that the inner mandrel of the suture cutting devices described herein can be configured to move (and not move) in any of a number of ways. For example, the inner mandrel may move axially (e.g., distally and/or proximally) with the respect to the holding tube to capture the suture, as shown in FIGS. 2B-2D. In some cases, the inner mandrel may move by rotating with the respect to the holding tube to capture the suture. In some cases, the inner mandrel may rotate with respect to the holding tube without (e.g., substantially) moving axially (e.g., distally and/or proximally) with respect to the holding tube to secure the suture. In some cases, the inner mandrel is prevented from moving in a certain way. For example, the inner mandrel may be rotatable but not axially movable with respect to the holding tube, or vice versa.

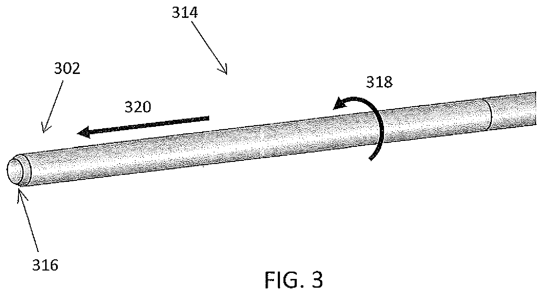

FIG. 3 illustrates exemplary motions of a cutter 314 as part of a suture cutting device, in accordance with some embodiments. Cutter 314 includes a distal end 302 having a cutting edge 316 (e.g., circular shaped circumference) that is configured to cut the suture. Cutter 314 can be configured to rotate 318 about a longitudinal axis when contacting the suture. The rotating motion 318 can reduce the force that the user needs to pull on the suture compared to a tube cutter that does not rotate. This is because the rotating motion 318 can provide a slicing force (tangential force) against the suture that can be applied using lower cutting forces compared to cutting only with a force in a normal direction with respect to the suture, such as an axial movement 320 in a distal direction toward the suture. In some embodiments, the cutter 314 is configured to only advance distally in an axial direction 320 toward the suture. In some embodiments, the cutter 314 is configured to only rotate 318. In some embodiments, the cutter 314 is configured to rotate 318 and advance in the distal axial direction 320. In some embodiments, the rotation 318 occurs simultaneously with the movement in the distal axial direction 320. For example, the cutter 314 can be configured to rotate 318 while advancing in the distal axial direction 320 to cut the suture. In some embodiments, the rotation 318 occurs separately (e.g., serially) with respect to the movement in the distal axial direction 320. In some examples, the cutter 314 is configured to advance 320 until it reaches the suture, then rotate 318 to cut the suture. In some cases, and the cutter 314 is advanced 320 for one period of time, and advanced in conjunction with rotation 318 for another period of time. That is, the rotating 318 and advancing 320 can occur in any combination.

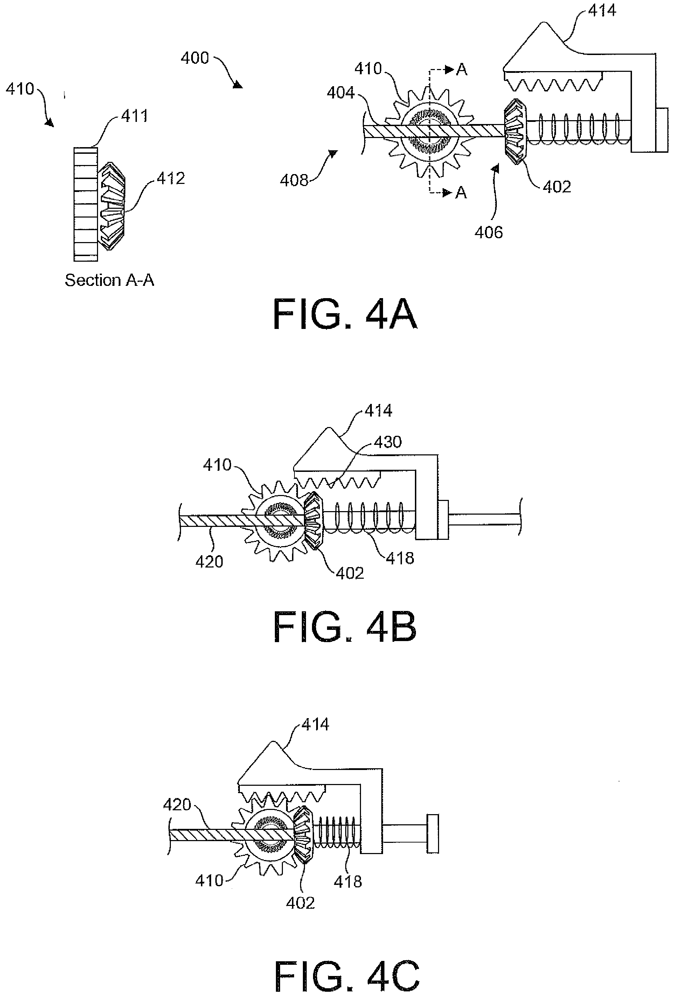

The rotating and/or axial motion of the cutters described herein can be accomplished using any of a variety of mechanisms. Such mechanisms can include a gear assembly, cam assembly, belt (and pulley) assembly and/or a rotor (e.g., motor) assembly. FIGS. 4A-4C illustrate an exemplary gear assembly 400 as part of a suture cutting device. The gear assembly 400 can include a first gear 402 (e.g., bevel gear) that is operationally coupled to a cutter 404 and an actuator 414. The first gear 402 can be coupled on a proximal end 406 of the cutter 404. A second gear 410 can have a first set of teeth 411 (e.g., spur gear portion) configured to engage with teeth 430 of the actuator 414, and a second set of teeth 412 (e.g., bevel gear portion) configured to engage with teeth of the first gear 402. The actuator 414 can be configured to cause the cutter 404 to move axially in a distal direction (advance) when activated by a user. For instance, the actuator 414 can be a slider button or switch that includes gear teeth 430 (e.g., on the bottom of the slider) that engage with the first set of teeth 411 on a second gear 410. Activation of the slider 414 can advance a proximal spring 418 and the first gear 402, which advances the cutter 404. The proximal spring 418, the first gear 402 and/or the cutter 404 can be coupled to a distal spring 420 that compresses and causes the first gear 402 to engage with the second gear 410. FIG. 4C shows the slider actuator 414 advanced farther (distally) by the user, which compresses the proximal spring 418 and causes the gear teeth 430 to rotate the second gear 410, which rotates the first gear 402 and the cutter 404. When the user releases the slider actuator 414, the proximal spring 418 extends and moves the slider actuator 414 in the proximal direction. The gear teeth 430 of the slider actuator 414 rotate the second gear 410 the opposite direction, which rotates first gear 402 and the cutter 404 in the opposite direction. The distal spring 420 extends, and the gear assembly 400 returns to its original state shown in FIG. 4A.

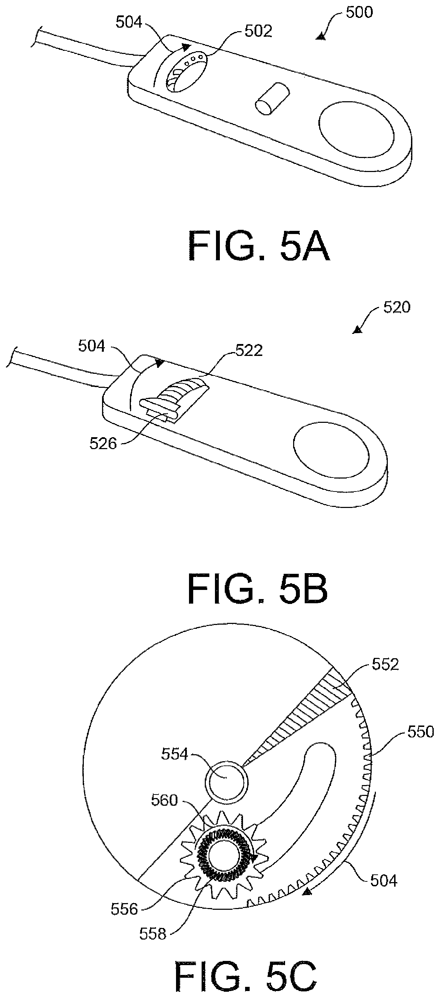

FIGS. 5A-5C show variations of actuators in the form of a wheel, which may be used in place of the slide actuator 414 in FIGS. 4A-4C. FIG. 5A shows a suture cutting device 500, which includes a wheel actuator 502. FIG. 5B shows a suture cutting device 520, which includes a wheel actuator 522 having an external knob 526 to provide extra leverage. The wheel actuator 500 or 520 can be activated by turning, such as by user's finger (e.g., thumb), in a direction 504. The section view of FIG. 5C shows how turning the wheel actuators 500 or 520 in the direction 504 can cause a first gear 550 to rotate in the direction 504. A thickness gradient 552 of the first gear 550 also causes the first gear 550 to translate along a gear axle 554 and engage with a second gear 556, thereby causing rotation of the second gear 556 and a cutter 558 that is coupled thereto. The translating motion of the first gear 550 (along axle 554) can also cause the cutter 558 to translate correspondingly. In this way, the rotating and translating of the first gear 550 can correspond to a rotating and translating motion of the cutter 558. In some embodiments, a distal spring could be used to return the wheel actuator back to its starting position, as described above with reference to FIGS. 4A-4C.

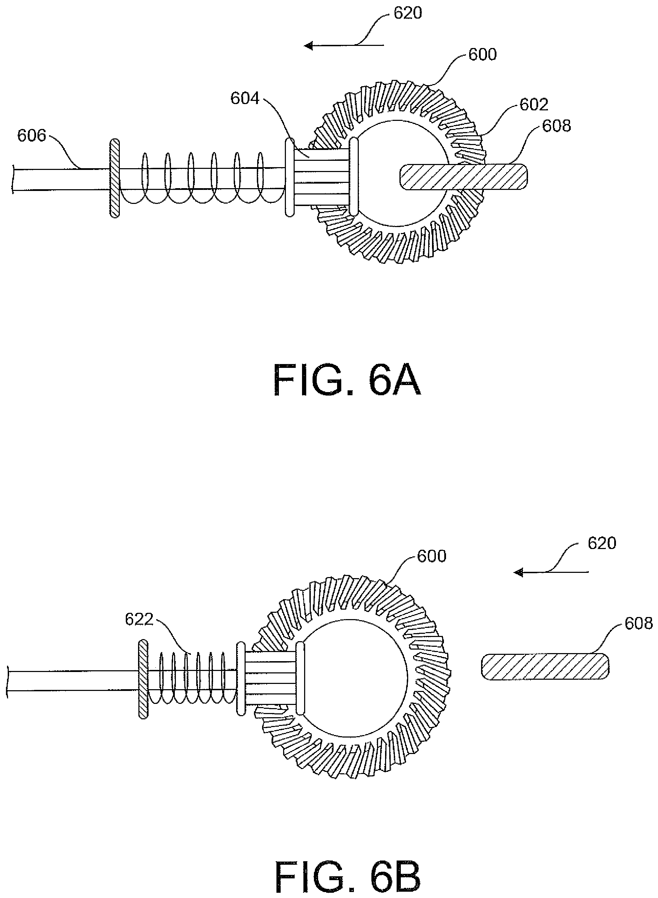

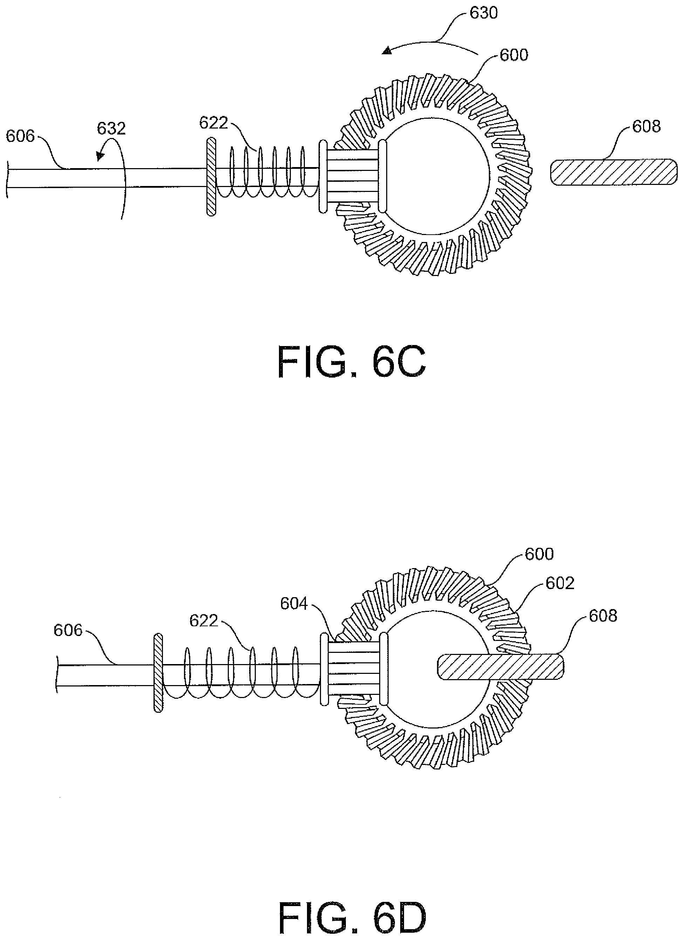

FIGS. 6A-6D illustrate an exemplary operation of another wheel actuator 600 configured to allow for separate rotation and advancement of a cutter 606. Wheel actuator 600 includes a side that has a set of teeth 602 that are configured to engage with a gear 604 that is coupled to a cutter 606. The teeth 602 of the wheel actuator 600 can be couple to a stop 608 (e.g., rib). FIG. 6A illustrates how when a user advances (e.g., pushes) the wheel actuator 600 in a distal direction 620, the cutter 606 also advances in the distal direction 610. The stop 608 can prevent the wheel actuator 600, gear 604 and cutter 606 from rotating. FIG. 6B shows the wheel actuator 600 after being advanced far enough such that the stop 608 disengages with the wheel actuator 600 and compresses a spring 622. Since the wheel actuator 600 is disengaged from the stop 608, the wheel actuator 600, gear 604 and cutter 606 are free to rotate. FIG. 6C illustrates how the user can apply a rotating (turning) motion 630 to the wheel actuator 600 that causes the cutter 606 to rotate 632 accordingly. After cutting the suture, the user can release the wheel actuator 600, causing the spring 622 to expand, and the wheel actuator 600, gear 604 and cutter 606 to retract proximally. As the wheel actuator 600 retracts, the wheel teeth 602 can reengage with the stop 608.

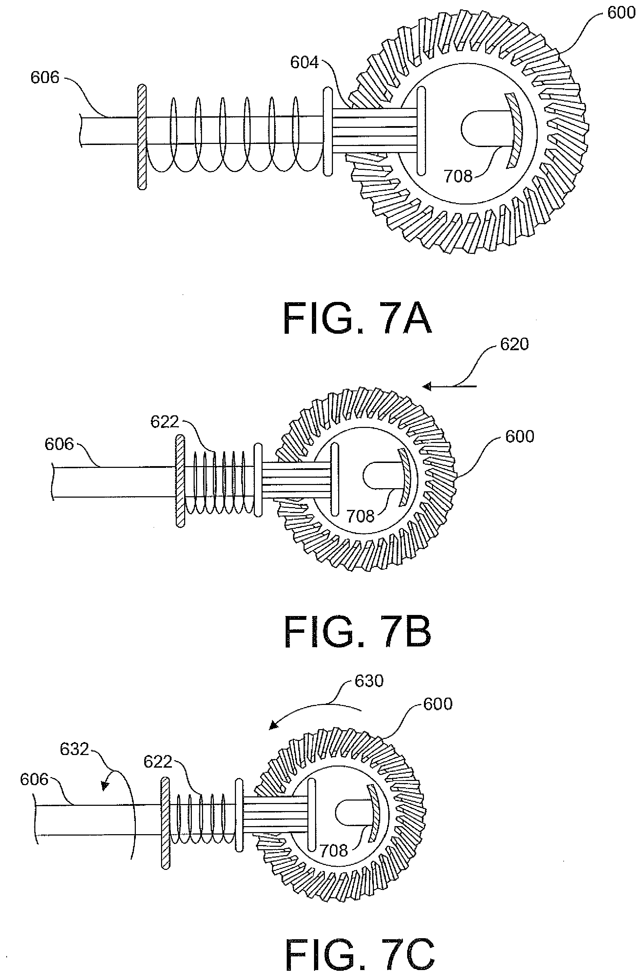

**Other** variations include mechanisms that make the wheel actuator more resistant to rotating than translating so that the wheel translates then rotates. An example is shown in FIGS. 7A-7C, which illustrate an exemplary operation of the wheel actuator 600 with a friction pad 708. When the user advances the wheel actuator 600 the cutter 606 in a distal direction 620, gear 604 turns and causes spring 622 to compress before friction pad 708 allows the wheel actuator 600 to rotate, as shown in FIG. 7B. After the wheel actuator 600 stops advancing, the user can overcome the force from the friction pad 708 to rotate 630 the wheel actuator 600 and rotate 632 the cutter 606, as shown in FIG. 7C.

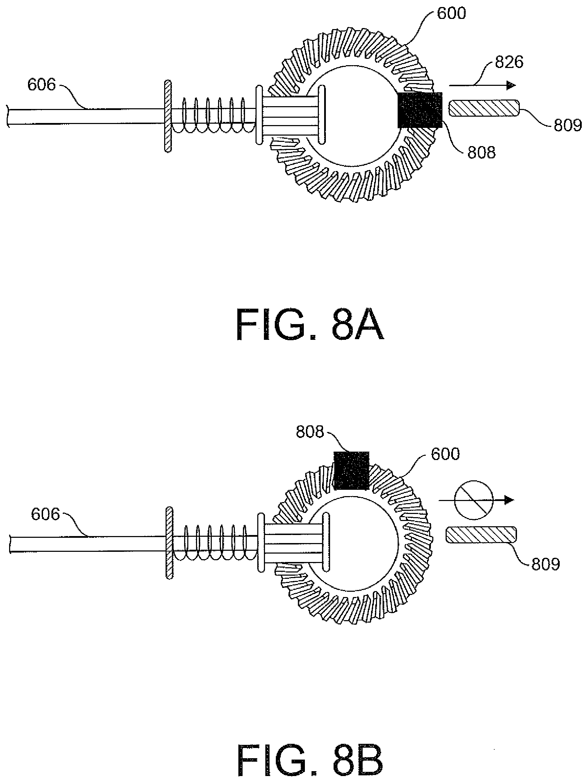

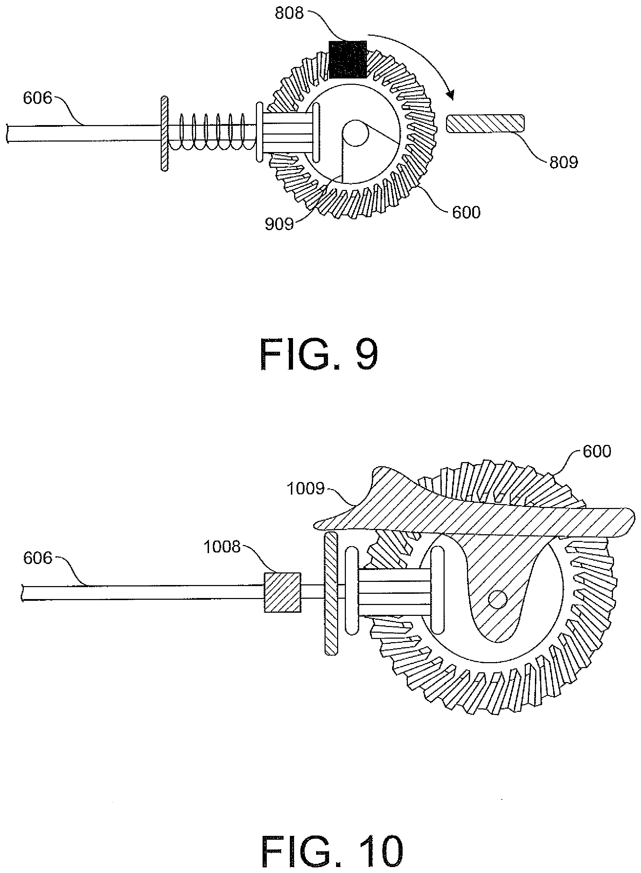

Additional wheel actuator variations include a catch that keeps the wheel actuator and cutter advanced making it easier for the user to rotate the wheel actuator multiple times. This could also make it easier for the user to rotate the wheel actuator in either or both directions, if desired. FIGS. 8A and 8B illustrates such an example. The wheel actuator 600 can include one or more openings 808 configured to engage with one or more corresponding catches 809. When the opening(s) 808 is/are aligned with the catch(es) 809, the wheel actuator 600 can retract proximally 826, as indicated in FIG. 8A. When the opening(s) 808 is/are not aligned with catch(es) 809, the wheel actuator 600 cannot retract proximally and the user can continue to rotate the wheel actuator 600 and cutter 606, as indicated in FIG. 8B. After cutting the suture, the user can release the catch(es) 809 to automatically allow the wheel actuator 600 and cutter 606 to retract. FIG. 9 shows another variation of wheel actuator 600 having a torsion spring 909 that rotates the wheel actuator 600 to automatically align the opening(s) 808 with catch(es) 809 after the user releases the wheel actuator 600. FIG. 10 shows a further variation of wheel actuator 600 with a friction element 1008 that keeps the cutter 606 advanced distally. The user can manually return the wheel actuator 600 and cutter 606 to their proximal positions. A slideable member 1009 may be operationally coupled to the wheel actuator to make it easier for the user to advance and retract the wheel actuator 600 and cutter 606.

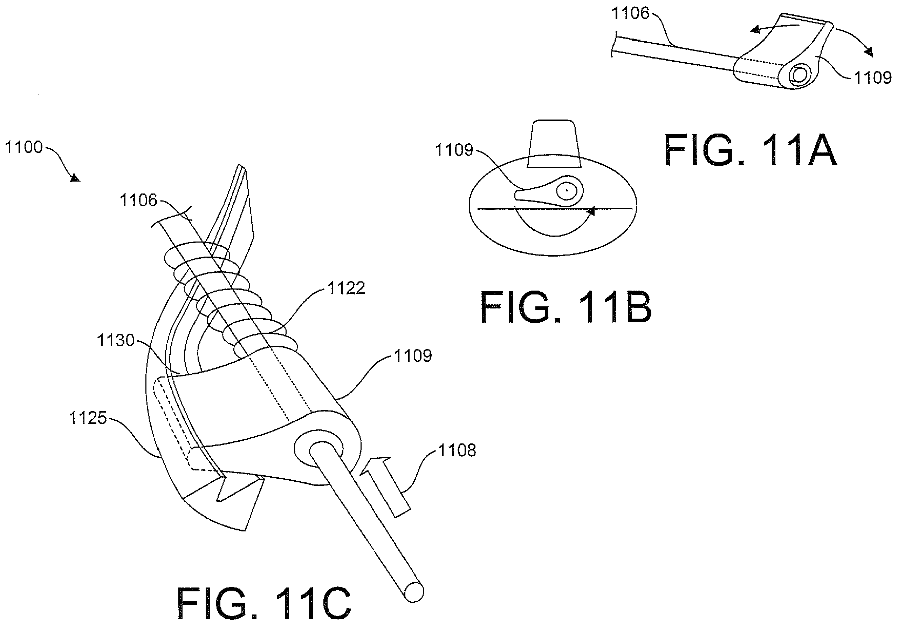

In some embodiments, a cam assembly is used to rotate the cutter. FIGS. 11A-11C illustrate an exemplary cam assembly 1100, where cutter 1106 is coupled to and configured to rotate with a follower cam 1109. As the user pushes an actuator (e.g., on the handle of the suture cutting device) in a distal direction 1108, the follower cam 1109 moves distally and rotates through a cam path 1130 defined by track 1125 causing the cutter 1106 to advance and rotate. The cam assembly 1100 may include a compression spring 1122 that compresses during the advancement of the follower cam 1109 and the cutter 1106 in the distal direction 1108, and expands to return the follower cam 1109, cutter 1106 and actuator to their proximal positions.

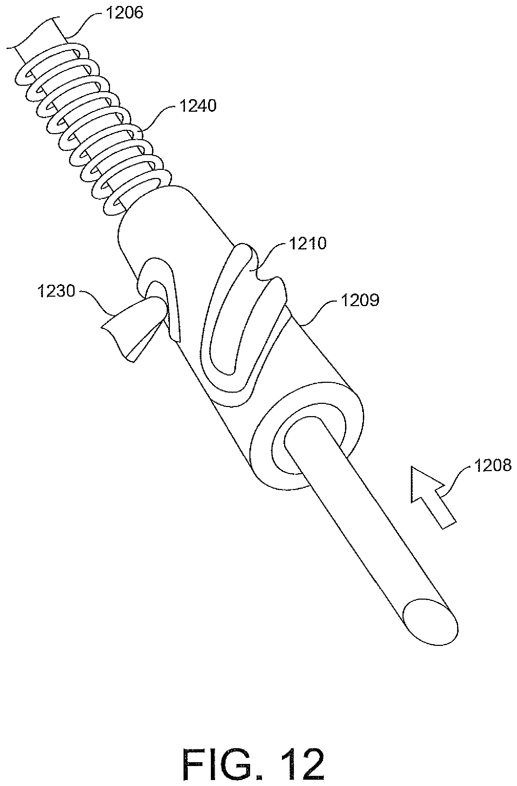

In some embodiments, a threaded collar assembly is used to rotate the cutter, such as the example shown FIG. 12. A threaded collar 1209 is coupled to and configured to rotate cutter 1206. The threaded collar 1209 includes a thread 1210 that is configured to engage with a tooth 1230 as part of the threaded collar assembly. As the user pushes an actuator (e.g., on the handle of the suture cutting device) in a distal direction 1208, the threaded collar 1209 moves distally, and tooth 1230 engages with and rotates the threaded collar 1209 causing the cutter 1206 to advance and rotate. The mechanism may include a compression spring 1240 that extends to return the threaded collar 1209, the cutter 1206 and the actuator to their proximal positions.

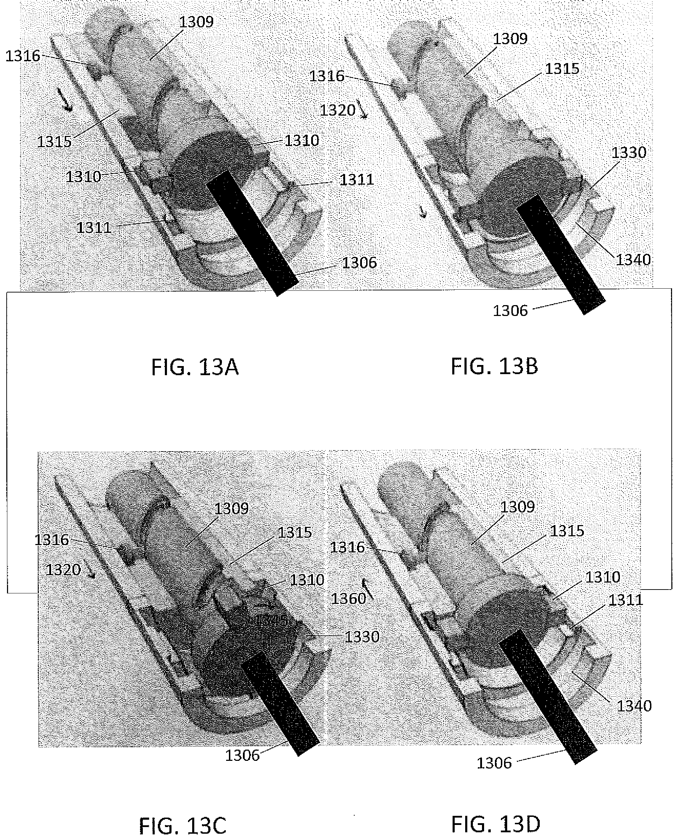

FIGS. 13A-13D illustrate an exemplary operation of another threaded collar assembly, which includes a threaded collar 1309 attached to cutter 1306, and an actuator 1315 attached to a pin 1316. In some embodiments, the actuator 1315 and the pin 1316 are a single part. The collar 1309 can include flanges 1310 that are configured to slide on ledges 1311. FIG. 13B shows the assembly 1300 after the user advances the actuator 1315 and pin 1316 in a distal direction 1320, which advances the collar 1309 and the cutter 1306. The flanges 1310 can slide on ledges 1311 to prevent the collar 1309 and cutter 1306 from rotating until the flanges 1310 enter a groove 1330 and contact stop surface 1340. FIG. 13C show the actuator 1315 and pin 1316 continued to be advanced where the distal movement of the actuator 1315 and pin 1316 cause the collar 1309 and the cutter 1306 to rotate 1345. The rotation 1345 causes the flanges 1310 to rotate in the groove 1330. At FIG. 13D, the user releases the actuator 1315 causing a spring to move the actuator 1315 proximally 1360. The flanges 1310 contact the stop surface 1340, and proximal movement of the actuator 1315 and pin 1316 cause the collar 1309 and cutter 1306 to rotate the opposite direction. The flanges 1310 rotate to align with ledges 1311, allowing the collar 1309 and cutter 1306 to retract proximally 1360 with the actuator 1315.

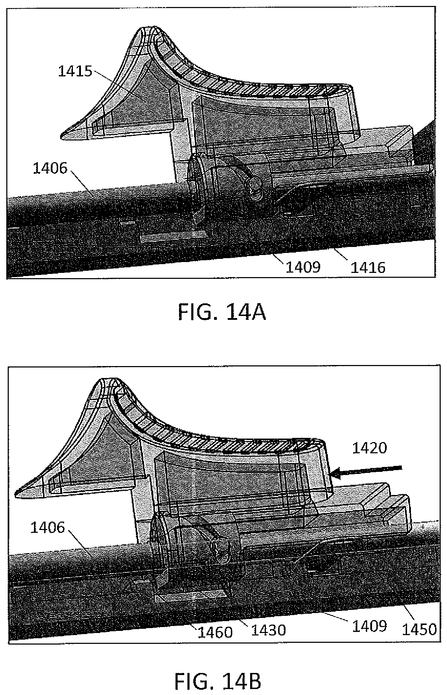

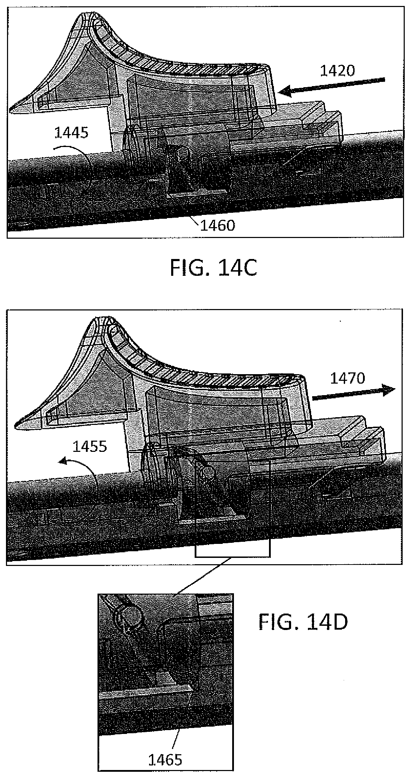

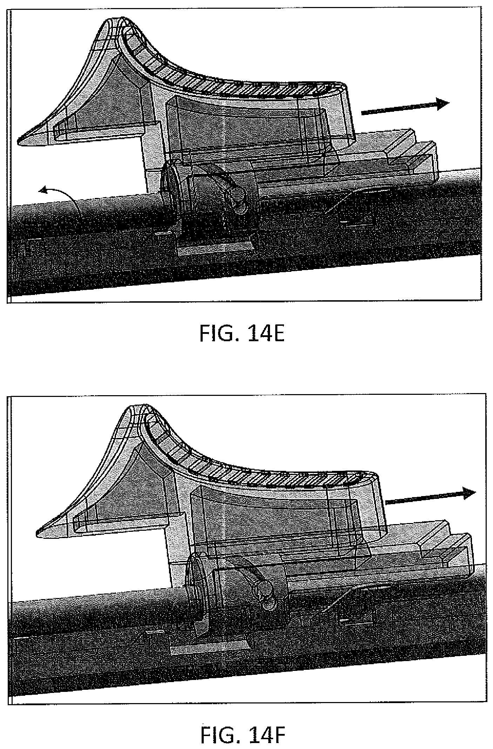

FIGS. 14A-14F illustrate an exemplary operation of a further threaded collar assembly, which includes a threaded collar 1409 attached to cutter 1406, and an actuator 1415 attached to a pin 1416. In some embodiments, the actuator 1415 and the pin 1416 are a single part. At FIG. 14B, the user advances the actuator 1415 (and the pin 1416) distally 1420, which advances the collar 1409 and the cutter 1406. The collar 1409 can include a flat surface 1430 that slides along a flat surface of a handle 1450 of the suture cutting device, thereby preventing the collar 1409 and the cutter 1406 from rotating until the flat surface 1430 of the collar 1409 disengages from the corresponding flat surface of the handle 1450 and the collar 1409 contacts a stop surface 1460. At FIG. 14C, the actuator 1415 and the pin 1416 continue advancement in the distal direction 1420. Stop surface 1460 prevents additional advancement of the collar 1409 and the cutter 1406, and the distal movement of the actuator 1415 and pin 1416 cause the collar 1409 and the cutter 1406 to rotate in a first direction 1445. At FIG. 14D, the user releases the actuator 1415 causing a spring to move the actuator 1415 proximally 1470. The collar 1419 can contact a second stop surface 1465 of the handle 1450. Proximal movement of the actuator 1415 and pin 1416 can cause the collar 1409 and the cutter 1406 to rotate a second direction 1455 opposite the first direction. At FIG. 14E, the flat surface 1430 of the collar 1409 rotates to align with the corresponding flat surface of the handle 1450, allowing the collar 1409 and the cutter 1406 to retract proximally 1470 with the actuator 1415. At FIG. 14E, the collar 1409 and the cutter 1406 retract proximally 1470 with the actuator 1415.

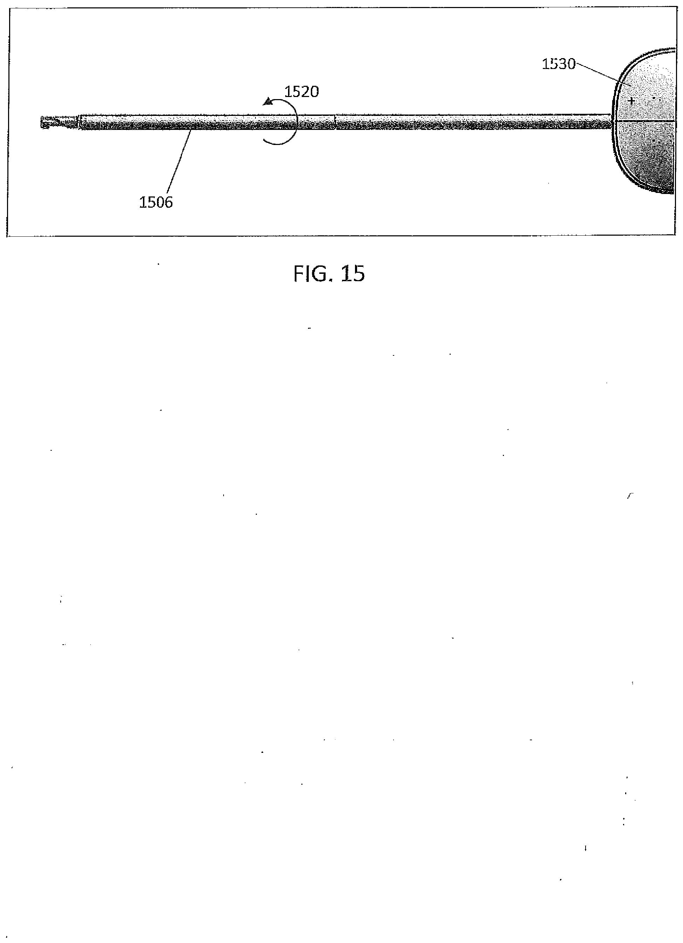

**In** some embodiments, the suture cutting device includes an actuator that stores potential energy, such as shown in the example of FIG. 15. The cutter 1506 can rotated 1520 via an actuator 1530 to generate potential energy. A catch keeps the cutter 1506 in the rotated position and stores the potential energy. After the cutter 1506 is advanced to the suture, the catch and potential energy are released to rotate the cutter 1506 and cut suture. In some embodiments, the potential energy is in the form of electrical energy (e.g., stored in a battery). In some embodiments, the potential energy is in the form of mechanical energy (e.g., stored in a spring or other elastic object).

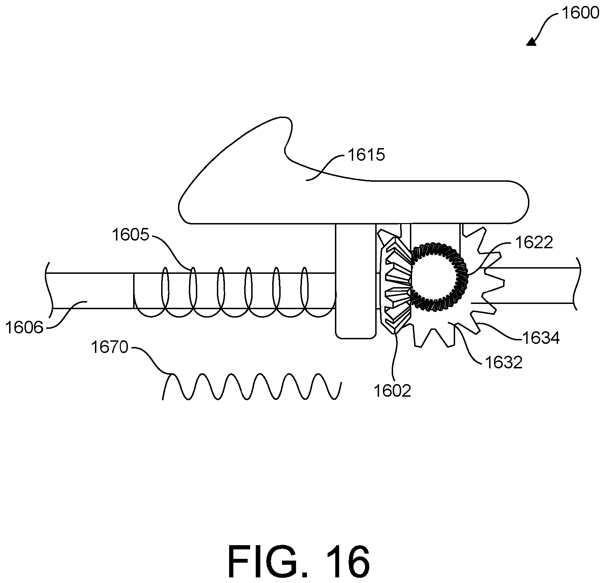

FIG. 16 shows another gear assembly 1600 having a first gear 1602 (e.g., bevel gear) operationally coupled to a cutter 1606, and a second gear 1622 (e.g., bevel gear) operationally coupled to a gear 1632. The first gear 1602 can include engagement features (e.g., teeth) that engage with corresponding engagement features (e.g., teeth) of the second gear 1622 such that rotation of the first gear 1602 can cause rotation of the second gear 1622. An actuator 1615 can be configured to keep both gears 1602 and 1622 engaged. When the user advances the actuator 1615 to advance the cutter 1606, the teeth of a rack 1670 (e.g., on the handle of the suture cutting device) can engage with corresponding teeth 1634 of a third gear 1632. In some embodiments, the second gear 1622 and the third gear 1632 are a single part. Continued advancement of the actuator 1615 and cutter 1606 can cause the third gear 1632, second gear 1622, first gear 1602 and the cutter 1606 to rotate. When the user releases the actuator, a spring 1605 can extend and retract the actuator 1615 and the cutter 1606 to their starting positions. In another variation, the length of the rack 1670 can be extended so that the third gear 1632 can engage with the rack 1670 for the full range of actuator 1615 travel.

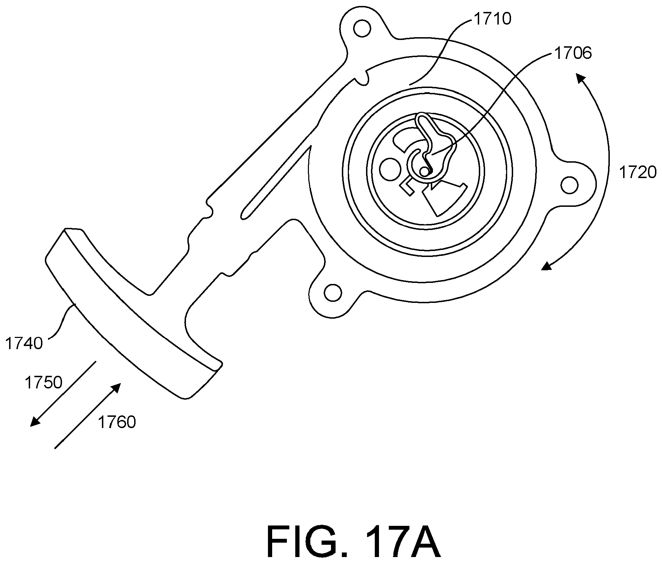

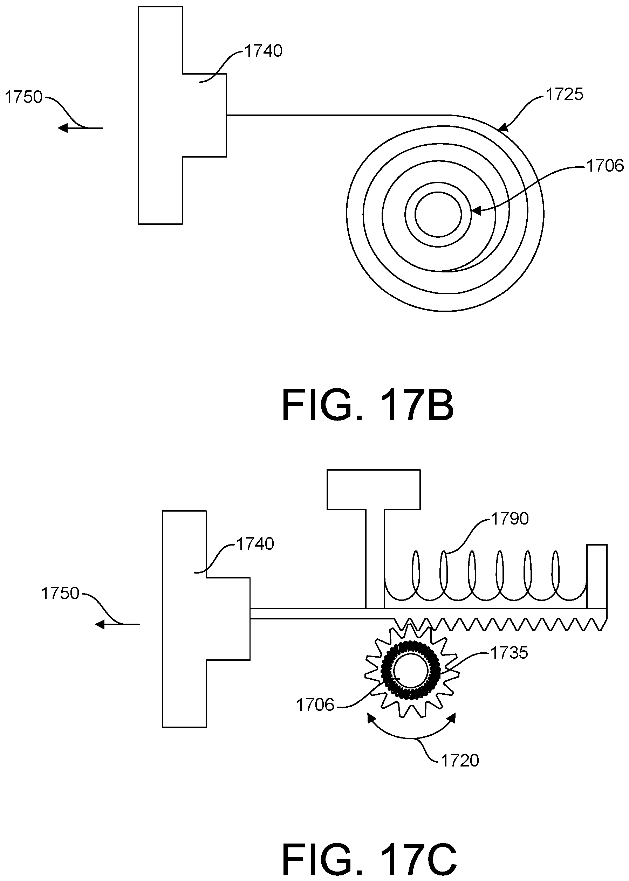

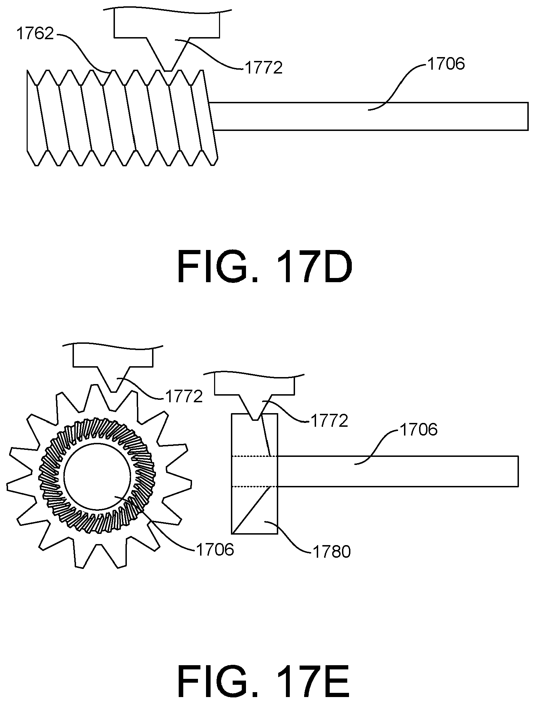

FIG. 17A-17E illustrate exemplary portions of suture cutting devices where the cutter is an axle (or is coupled to an axle) of a wheel. In FIG. 17A, the cutter 1706 can be coupled to a wheel 1710 such that when the user pulls 1750 a handle 1740, the wheel 1710 rotates 1720 and advances distally causing cutter 1706 to rotate and advance correspondingly. When the user releases the handle 1740, the handle 1740 may retract 1760 to its starting position. Alternately, the user can manually return the handle 1740 it to its starting position. FIG. 17B shows a variation where the wheel 1710 can be operationally coupled to a spring 1725 (e.g., flat coil spring), and spring 1725 can be operationally coupled to the handle 1740. When the user pulls handle 1740, the spring 1725 can unwind and rotate the wheel 1710 and cutter 1706. When the user releases the handle 1740, the spring 1725 can wind and rotate the 1710 and cutter 1706 the opposite direction. Alternately, the cutter 1706 can be connected directly to the spring 1725, eliminating the wheel 1710. FIG. 17C shows a variation where the cutter 1706 is coupled to a gear 1735, which can be configured to engage with the teeth of a rack 1742. The rack 1742 can be coupled to the handle 1740. When the user pulls the handle 1740, the rack 1742 can cause the gear 1735 and cutter 1706 to rotate and cause a spring 1790 to compress. When the user releases the handle 1740, the spring 1725 can extend and actuate the rack 1742 in the opposite direction, causing the gear 1735 and cutter 1706 to rotate the opposite direction. As a variation, the spring 1790 may be removed and the user can manually return the handle 1740 to its starting position. In some embodiments, the cutter 1706 is configured to advance with its rotation, as shown in FIG. 17D and 17E. FIG. 17D shows a variation where the cutter 1706 engages with a threaded body 1762. As the cutter 1706 and the threaded body 1762 rotate, one or more teeth 1772 causes the threaded body 1762 to advance, thereby advancing the rotating cutter 1706. FIG. 17E shows a variation where the cutter 1706 is engaged with a wheel 1780. Wheel 1780 can include a perimeter surface with a thickness gradient (e.g., around the circumference of the wheel 1780) that is configured to engage with the one or more teeth 1772. As the cutter 1706 and wheel 1780 rotate, the gradient around the circumference of the wheel 1780 can cause the wheel 1780 and cutter 1706 to advance distally.

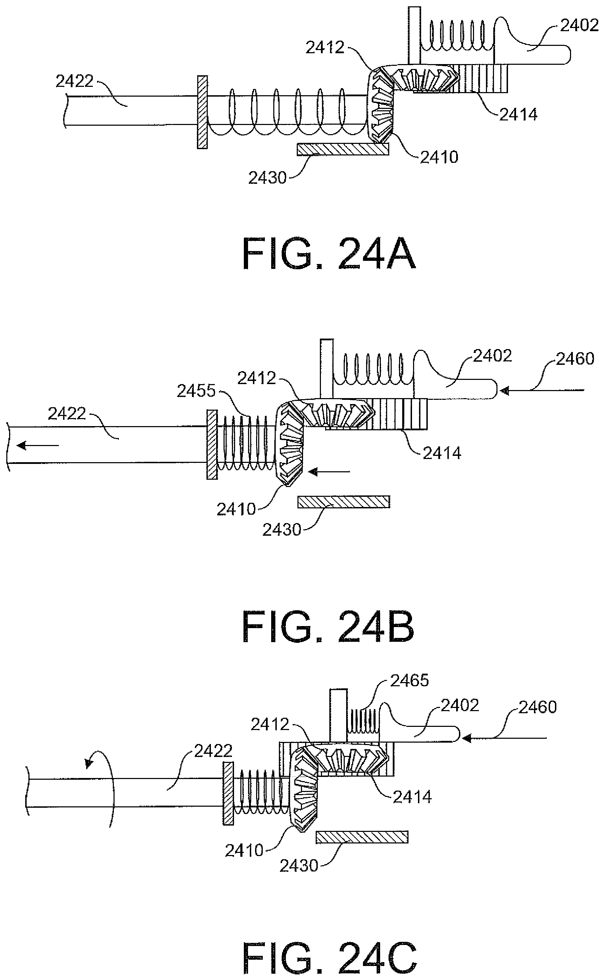

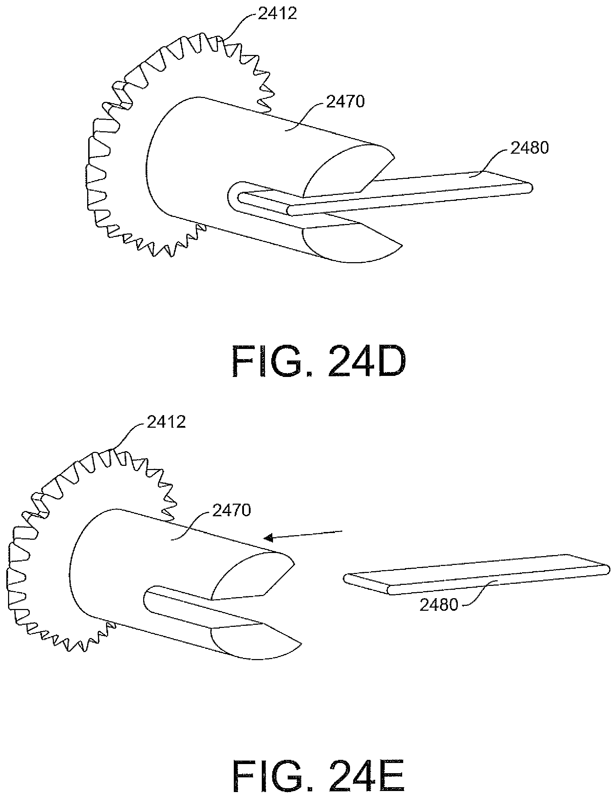

FIGS. 24A-24E show another variation of an exemplary gear assembly as part of a suture cutting device. The gear assembly of FIGS. 24A-24C may be variations of the gear assemblies shown in FIGS. 16 and/or 17A-17E. FIG. 24A shows a first gear 2410 (e.g., bevel gear) coupled to the cutter 2422 and configured to engage with a second gear 2412 (e.g., bevel gear). A rack 2414 can be coupled to the actuator 2402 and configured to engage with the second gear 2412. FIG. 24A shows the gear assembly at a first stage, when the teeth of the first gear 2410 are engaged with a rib 2430 to preventing the gears 2410 and 2412 from spinning. FIG. 24B shows the gear assembly at a second stage when the user actuates the actuator 2402 (e.g., advances the actuator 2402 in the distal direction 2460), causing the gears 2410 and 2412 and the cutter 2422 to advance and a first spring 2455 to compress. The first gear 2410 disengages from rib 2430, allowing the gears 2410 and 2412 and cutter 2422 to rotate. FIG. 24C shows the gear assembly at a third stage, when user continues to actuate the actuator 2402, causing a second spring 2465 to compress and the rack 2414 to rotate the gears 2410 and 2412 and cutter 2422. When the user releases the actuator 2402, the second spring 2465 can extend, causing the actuator 2402 and the rack 2414 to retract proximally. This can cause the gears 2410 and 2412 and cutter 2422 to rotate the opposite direction. Then, the first spring 2455 can extend, causing the gears 2410 and 2412 and cutter 2422 to retract proximally. The teeth of the first gear 2410 can engage with the rib 2430 as the first gear 2410 retracts proximally. FIGS. 24D and 24E show a variation where instead of a rib 2430, the second gear 2412 includes a slotted post 2470 that is configured to engage with a flange 2480. For example, when the actuator 2402 and the gears 2410 and 2412 are retracted in the proximal position, the slotted post 2417 can engage with the flange 2480 to prevent the gears 2410 and 2412 and cutter 2422 from rotating when retracted in the proximal position (FIG. 24D). When the actuator 2402 is advanced distally, the slotted post 2417 can disengage from the flange 2480, allowing the gears 2410 and 2412 and cutter 2422 to rotate (FIG. 24E).

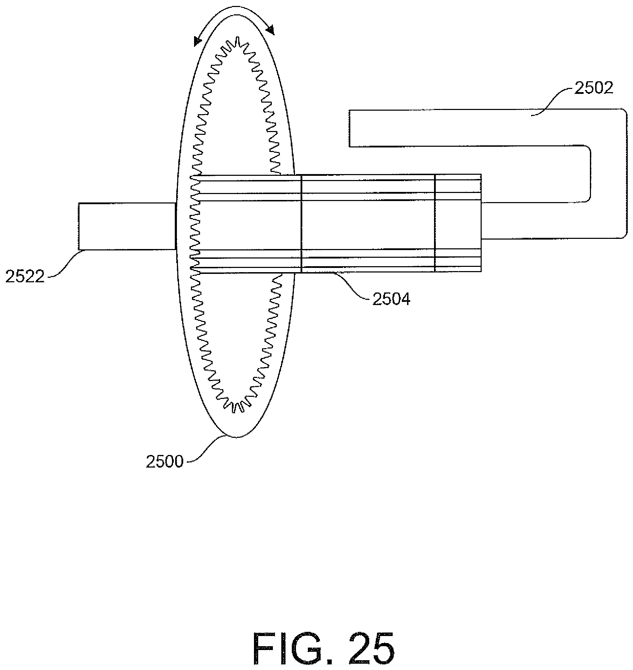

FIG. 25 shows another variation of a gear assembly, which includes a gear 2504 is coupled to the cutter 2522. The teeth on the gear 2504 can be configured to engage with the teeth on a wheel 2500. When the user actuates (e.g., advances distally) actuator 2502, the gear 2504 and cutter 2522 advance distally. When the gear 2504 advances, the teeth of the wheel 2500 stay engaged with the teeth of the gear 2504. When the user rotates the wheel 2500, this causes the gear 2504 and cutter 2522 to rotate. After the suture is cut, the user can release a catch to automatically allow the actuator 2502 and cutter 2522 to retract proximally, or the user can manually return the actuator 2502 to its proximal position.

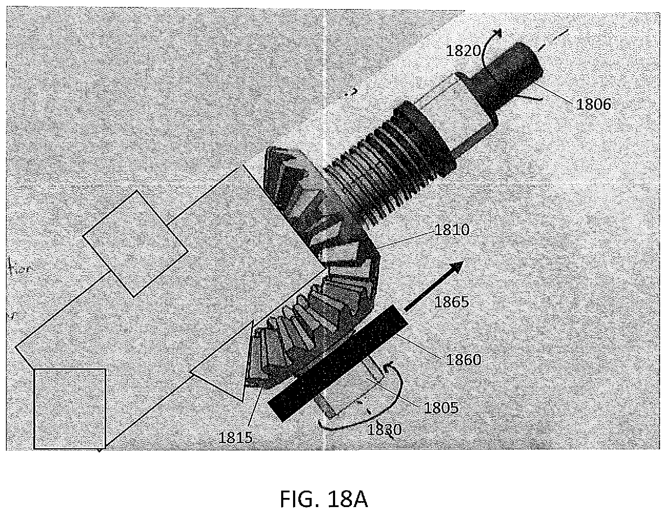

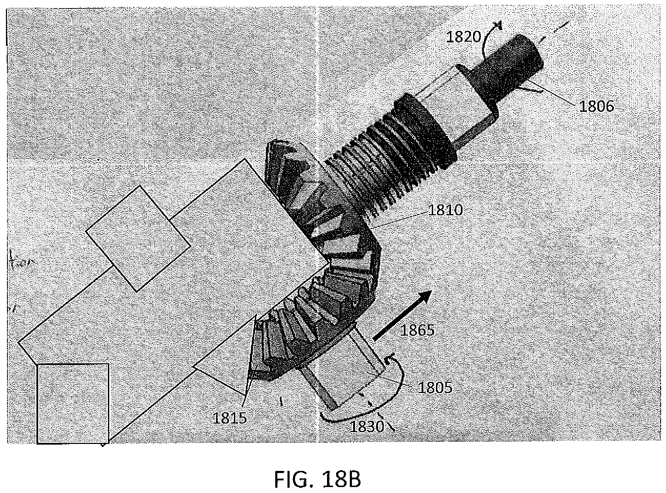

FIGS. 18A and 18B show examples of a cutter that is operationally coupled with a twist knob. FIG. 18A shows a cutter 1806 coupled to a first gear 1810 (e.g., bevel gear), which is coupled to a twist knob 1805. An actuator 1860 engages with a second gear 1815 (e.g., bevel gear) and operationally coupled to the twist knob 1805 so that when the user advances 1865 the actuator 1860, the cutter 1806 also advances. When the user rotates 1830 the twist knob 1805, the second gear 1815 rotates the first gear 1810, thereby rotating 1820 the cutter 1806. In some embodiments, the twist knob 1805 is rotated 1830 about an axis that is orthogonal to the axis of rotation 1820 of the cutter 1820. FIG. 18B shows a variation where the cutter 1806 is connected to the second gear 1815 is coupled to the twist knob 1805 (without an actuator). The user advances 1865 the twist knob 1805, which advances the cutter 1806. When the user rotates 1830 the twist knob 1805, the second gear 1815 rotates first gear 1810, thereby rotating the cutter 1806.

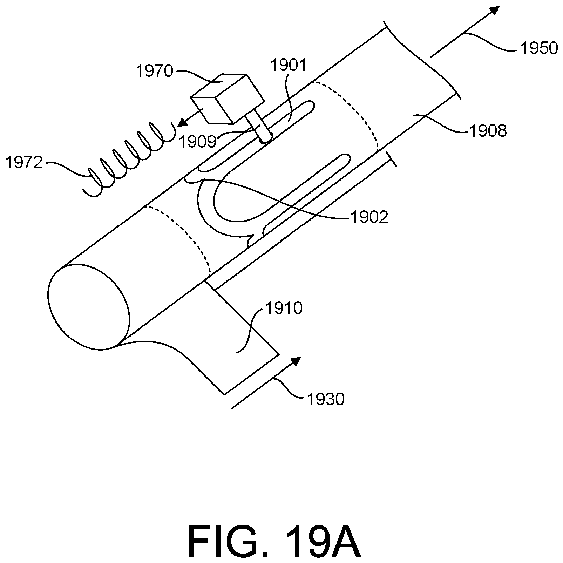

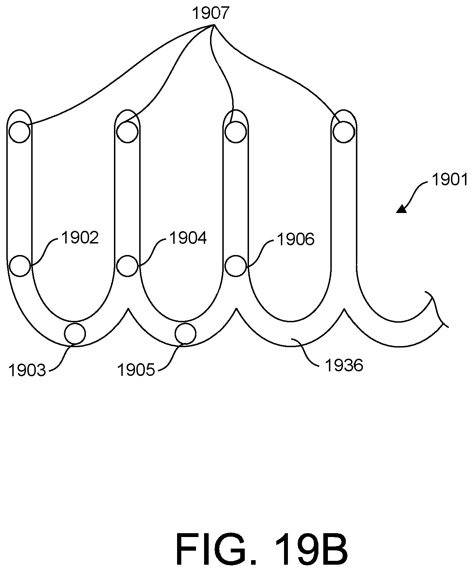

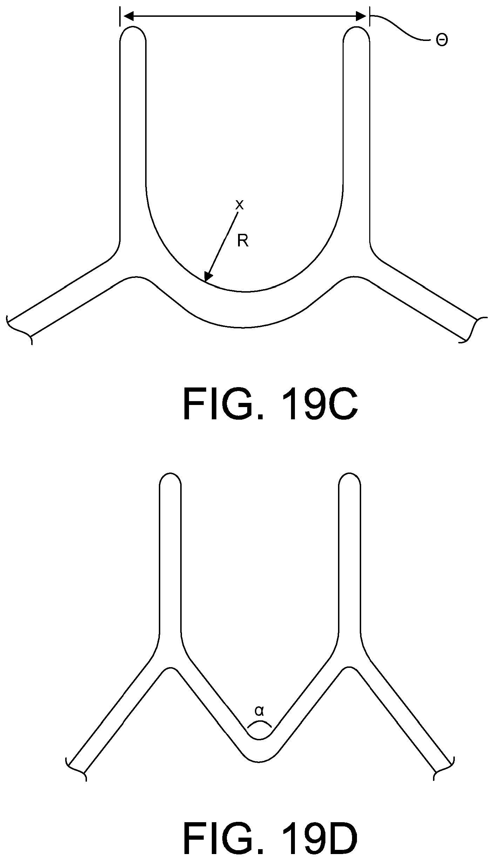

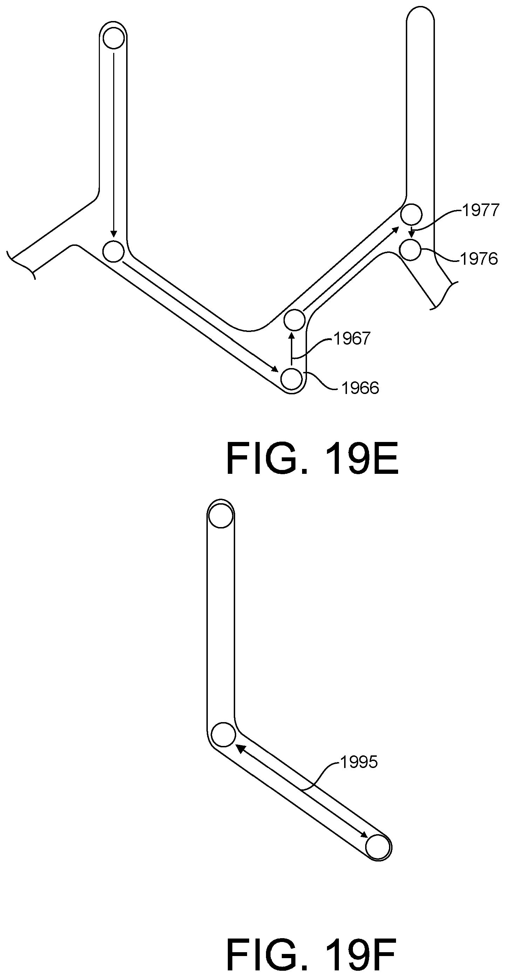

In some embodiments, motion of the cutter is at least partially defined by a guide path. FIGS. 19A-19F show an exemplary variations of guide paths. FIG. 19A shows a guide path 1901, which may be a channel (e.g., grove) that is cut in or on the cutter, or in or on a component coupled to the cutter 1908. The guide path 1901 can define the length of travel of the cutter 1908. The guide path 1901 may be part of a barrel cam. A pin 1909 can reside in the guide path 1901 and be coupled to an actuator 1910. In some embodiments, the pin 1909 is coupled to a slider 1970 that is coupled to a spring 1972. When the user advances the actuator 1910 distally 1930, the cutter 1908 and guide path 1901 can also advance distally 1950. The user may advance the actuator 1910 distally until the pin 1909 reaches a first guide path location 1902. A catch can be used to keep the cutter 1908 in the distally advanced position. In some embodiments, the guide path 1901 provides rotation in only one direction when the cutter 1908 is advanced. FIG. 19B illustrates different locations along the guide path 1901. The actuator 1910 can proximally cause the spring 1972 to compress and cause the pin 1909 to rotate the guide path 1901 and cutter 1908. This puts the pin 1909 at a second guide path location 1903. When the user releases actuator 1910, the spring 1972 extends and moves the actuator 1910 distally, causing the pin 1909 to further rotate the guide path 1901 and cutter 1908. This puts the pin 1909 at third guide path location 1904. The user continues to actuate and release the actuator 1910, causing the pin 1909 to move the guide path 1901 to locations 1905, 1906, etc., thereby rotating the guide path 1901 and cutter 1908 until the suture is cut. Then, the user can release the catch to automatically allow the actuator 1910 to retract proximally, or may manually return the actuator 1910 to its proximal position. When the actuator 1910 retracts, the guide path 1901 and cutter 1908 retract. This puts the pin 1909 at one of a number of retracted cutter positions 1907. In some embodiments, the guide path 1901 is in accordance with a pattern that repeats evenly (e.g., four times = four rotations of 90 degrees each). The pattern of the guide path 1901 may be changed to decrease the rotation angle for each activation of the slider 1970. The depth of the guide path 1901 (e.g., channel depth) may be varied to control movement of the pin 1909. For example, the depth of the guide path 1901 may be chosen such that the pin 1909 can only go in one direction (e.g., since the pin 1909 can be spring loaded). In some cases, the depth of the guide path 1901 decreases in depth along a "U" shaped section (e.g., section 1936). FIG. 19C shows how the guide path 1901 geometry can be varied to control cutter 1908 rotation angles Θ (e.g., 180 degrees) for each actuation/release of the actuator 1910. The radius of curvature R of the turns within the guide path 1901 may also vary. FIG. 19D a variation where the guide path 1901 includes angled turns (e.g., instead of curved turns) where the angle α of the angled turns can vary. FIG. 19E shows a variation of guide path 1901 where the geometry makes cutter 1908 rotation in one direction more reliable. For example, the guide path 1901 can include a first widened area 1966 that biases the movement of the pin 1909 in a first direction 1967 and/or a second widened area 1976 that bias the movement of the pin 1909 in a second direction 1977. FIG. 19F shows a variation of guide path 1901 having a geometry that can cause the cutter 1908 to oscillate when the actuator 1910 is actuated and released, in accordance with a back and forth direction 1995 of the pin 1909.

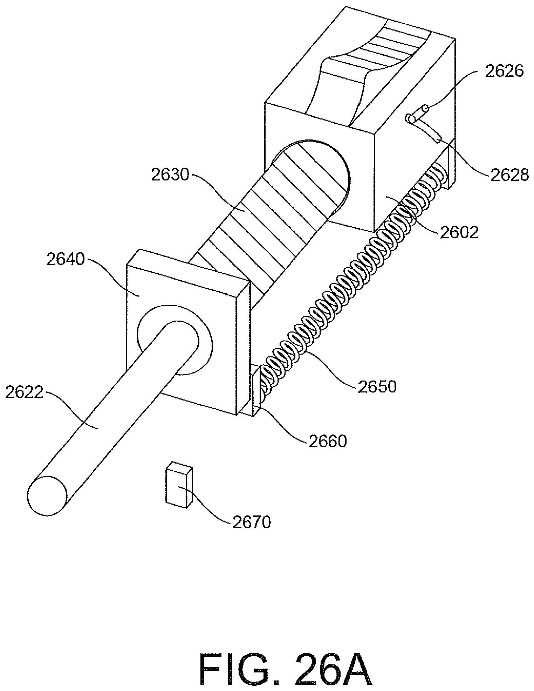

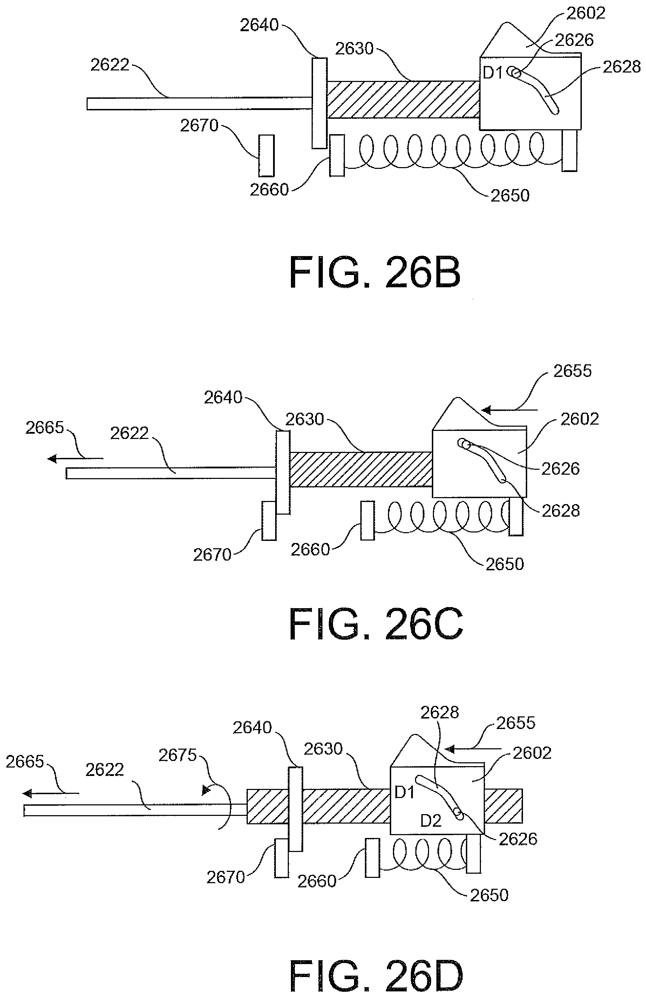

The guide path can be on any portion of the suture cutting device. FIGS. 26A-26D shows an exemplary suture cutting device having a guide path on an actuator 2602. The suture cutting device can include a threaded shaft 2630 that is rigidly coupled to cutter 2622 and pin 2626. The pin 2626 can be configured to reside in a path 2628, which may be in or on the actuator 2602 or in or on a component that is coupled to the actuator 2602. In some embodiments, the actuator 2602 may be activated by axially translating actuator 2602 (e.g., advancing distally). The path 2628 may be a channel (e.g., slot) for accommodating the pin 2626. In some embodiments, the path 2628 has a helical shape to provide corresponding rotation and translation (e.g., advancement) of the cutter 2622. Spring 2650 is operationally coupled with the actuator 2602. When the actuator 2602 is not actuated, a nut 2640 engaged with the threaded shaft 2630 can be in contact with a first stop 2660, as shown in FIG. 26B. The actuator 2602 is retracted in its proximal position and the pin 2626 is at a first location D1 in the path 2628. FIG. 26C illustrates when the user actuates the actuator 2602 (e.g., advances the actuator distally 2655) the pin 2626, the threaded shaft 2630, the nut 2640 and the cutter 2622 advance in a distal direction 2665 until the nut 2640 contacts a second stop 2670. FIG. 26D illustrates when the user can continue to actuate the actuator 2602 (e.g., advance the actuator further distally 2655) to cause one or more actions to occur. For example, the pin 2626 can move from the first location D1 to as second location D2 in the path 2628. The movement of the pin 2626 in the path 2628 can cause the threaded shaft 2630 to rotate. The rotation of the threaded shaft 2630 can cause the cutter 2622 to rotate 2675 and advance distally 2665 in accordance with a thread pitch on the threaded shaft 2630 (and nut 2640). When the user releases the actuator 2602, the spring 2650 can extend, causing multiple one or more actions to occur. For example, the actuator 2602 can retract proximally (opposite of distal direction 2655). The pin 2626, the threaded shaft 2630, the nut 2640 and the cutter 2622 can retract until the nut 2640 contacts the first stop 2660. The pin 2626 can move from the second location D2 to the first location D1 in the path 2628. The movement of the pin 2626 in the path 2628 can cause the threaded shaft 2630 to rotate the opposite direction. The rotation of the threaded shaft 2630 can cause the cutter 2622 to rotate and retract in accordance with the thread pitch on the threaded shaft 2630 (and nut 2640).

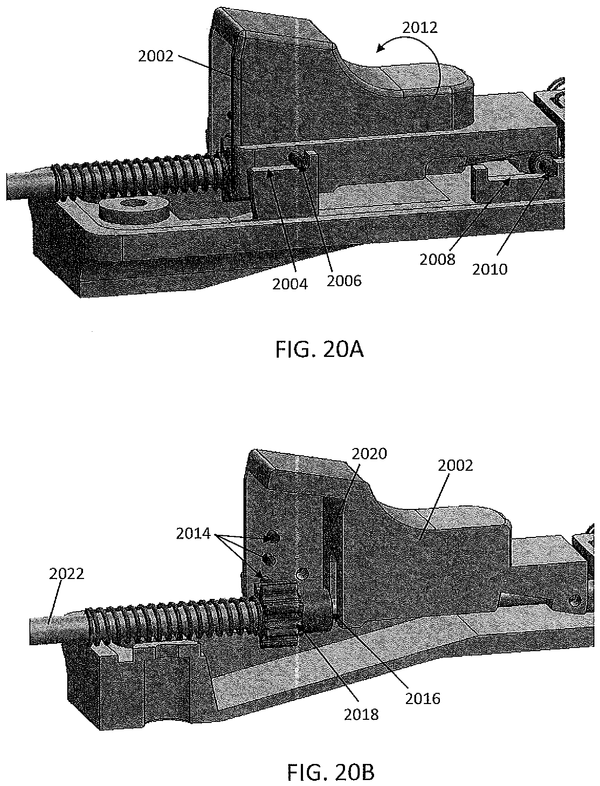

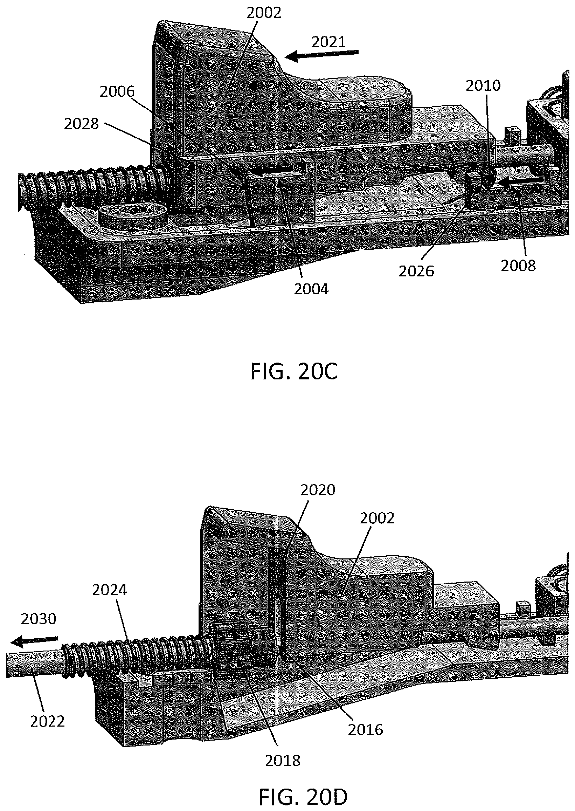

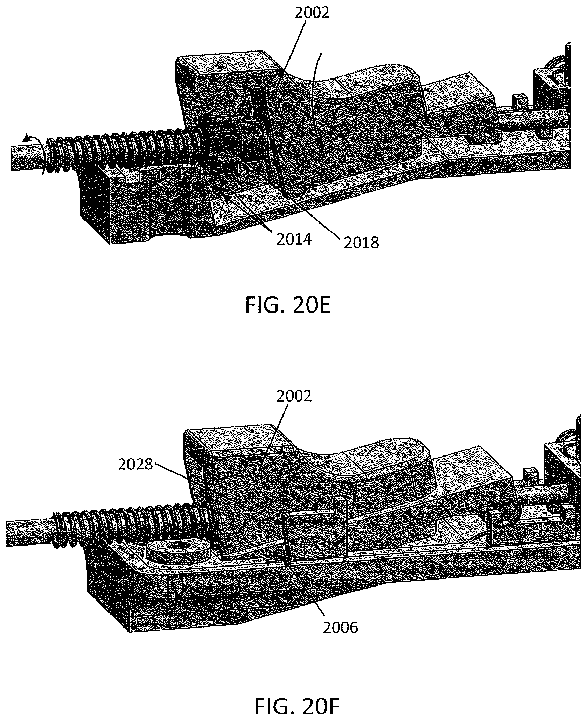

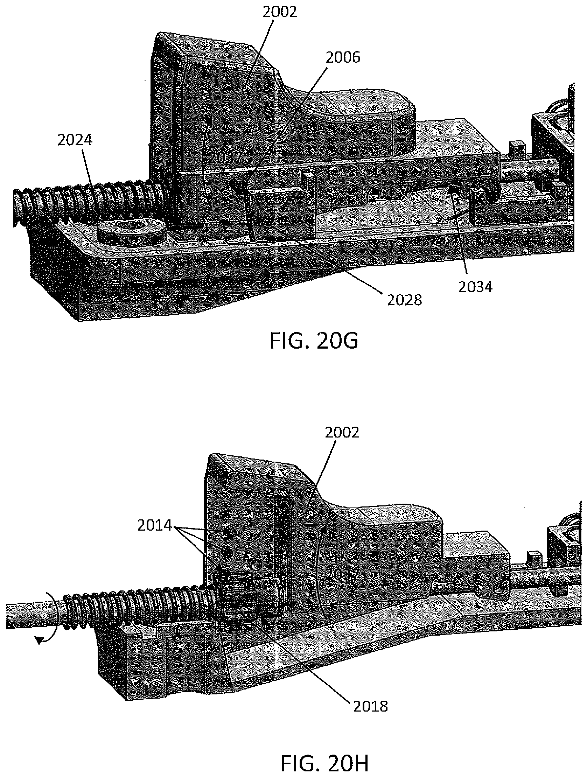

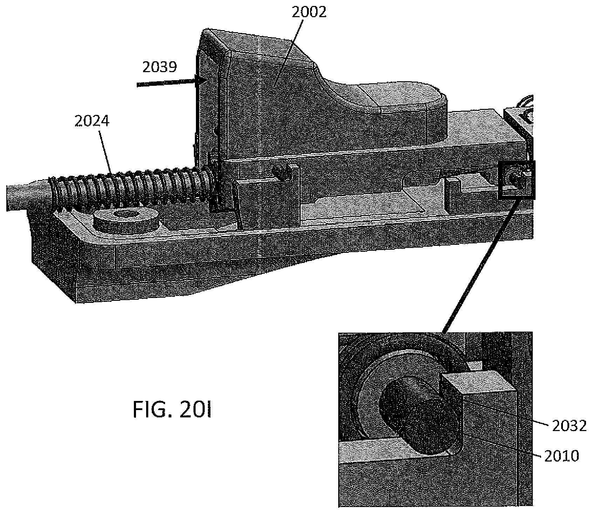

FIGS. 20A-20I illustrate an example operation of another exemplary gear assembly as part of a suture cutting device. FIG. 20A shows an actuator 2002 coupled to a first pin 2006 and a second pin 2010. The first pin 2006 rests on a first ledge 2004, and the second pin 2010 rests on a second ledge 2008. This geometry is mirrored on the opposite side 2012 of the actuator 2002. In some embodiments, the actuator 2002 and pins 2006 and 2010 are part of a single part. FIG. 20B shows a section view in showing the actuator 2002 can be coupled to actuator teeth 2014. In some embodiments, the teeth 2104 are on an internal surface of the actuator 2002. The actuator 2002 can have a first push surface 2020 that contacts a second push surface 2016 on a gear 2018. The gear 2018 can be coupled to a cutter 2022 and be configured to engage with actuator teeth 2014. FIG. 20C illustrates how when the user translates (e.g., slides) the actuator 2002 distally 2021, the pins 2006 and 2010 can slide on ledges 2004 and 2008, respectively, until the second pin 2010 contacts a first stop surface 2026 and the first pin 2006 is on a retaining surface 2028. The section view of FIG. 20D illustrates how when the actuator 2002 translates (e.g., slides) distally 2021, a spring 2024 can compress and push the first push surface 2020, causing the gear 2018 and the cutter 2022 to translate distally 2030. The section view of FIG. 20E shows the actuator 2002 after it has stopped translating (e.g., sliding) distally, it can be configured to rotate 2035 (e.g., pivot), such as in a downward direction. The actuator 2002 rotation 2035 can cause the actuator teeth 2014 to rotate the gear 2018 and the cutter 2022. FIG. 20F shows when the actuator 2002 rotates down, the retaining surface 2028 can prevent the first pin 2006 and the actuator 2002 from moving proximally. FIG. 20G shows when the user releases the actuator 2002, the energy stored in spring 2024 when compressed can cause the actuator 2002 to rotate 2037 back to its original position (e.g., upward). The first pin 2006 can disengage from (e.g., move above) the retaining surface 2028 so that the actuator 2002 can move proximally. The section view of FIG. 20H shows that when the actuator 2002 rotates 2037 (e.g., upward), the actuator teeth 2014 can rotate the gear 2018 and the cutter 2022 in the opposite direction. FIG. 20I shows that after the actuator 2002 has rotated into its original position (e.g., up), the spring 2024 can extend (decompress) and the actuator 2002 can move proximally 2039 until the second pin 2010 contacts a second stop surface 2032.

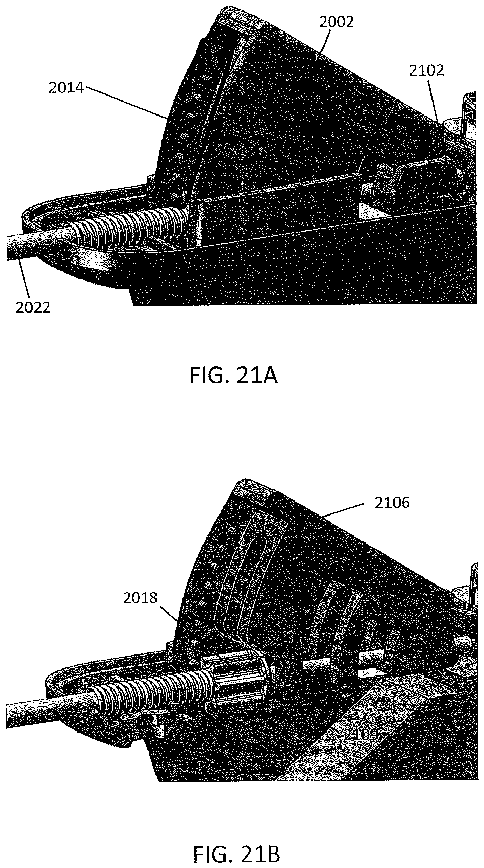

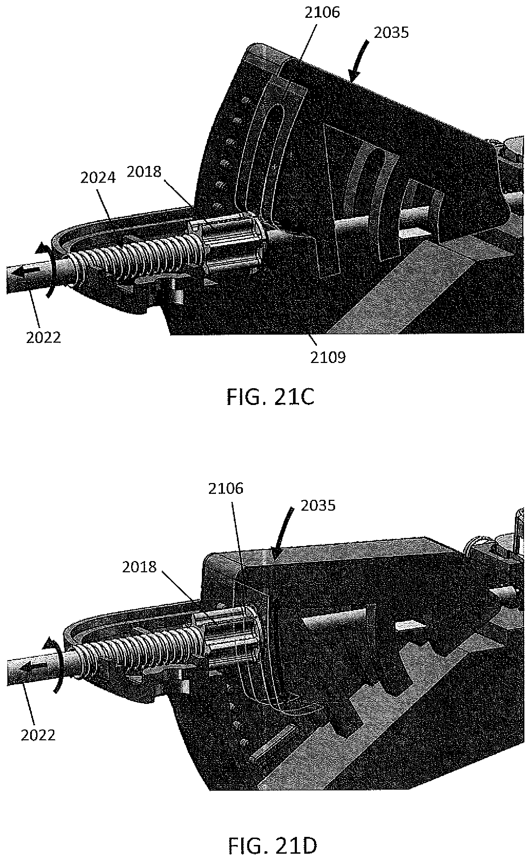

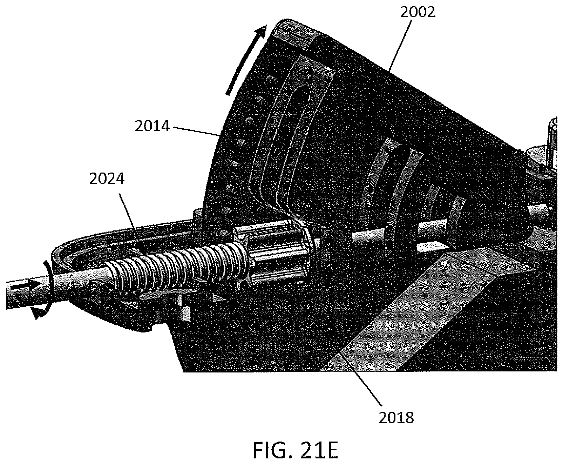

FIGS. 21A-21E illustrate an example operation of a variation of the exemplary gear assembly of FIGS. 20A-20F. FIG. 21A shows the actuator 2002, which has actuator teeth 2014, can be configured to rotate about an axle 2102 to rotate the cutter 2022. The section view of FIG. 21B shows how, in this variation, the actuator 2002 can have a cam surface 2106 that can be configured to contact a rim 2109 of the gear 2018. FIGS. 21C and 21D illustrate how when the user depresses the actuator 2002, the actuator 2002 can rotate (e.g., downward) about its axle and one or more of the following actions can (e.g., simultaneous) occur: 1) By engaging with the rim 2109 of the gear 2018, the cam surface 2106 can advance the gear 2018 and cutter 2022 distally; 2) the cutter 2022 advancement can be controlled by the geometry of a first section 2133 and/or a second section 2137 of a path of the cam surface 2106 (other variations may have a different cam path geometries); and 3) the first section 2133 of the cam path can advance the cutter 2022 at a first rate, and the second section 2137 of the cam path can advance the cutter 2022 at a second rate (which can be the same or different than the first rate). FIG. 21E shows that when the user releases the actuator 2002, a second spring can rotate the actuator 2002 back to the original position (e.g., up). The actuator teeth 2014 can rotate the gear 2018 and cutter 2022 the opposite direction, the spring 2024 can extend, and the gear 2018 and cutter 2022 can retract, as described above.

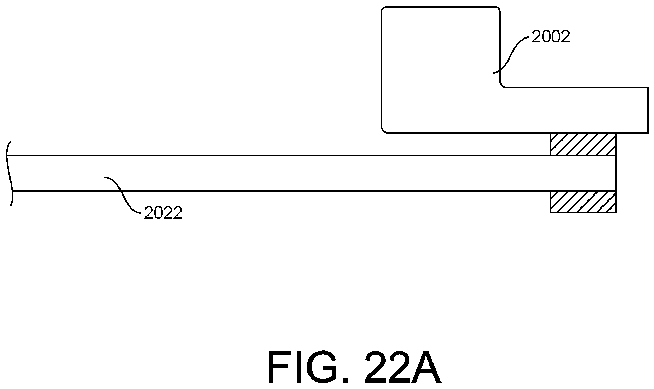

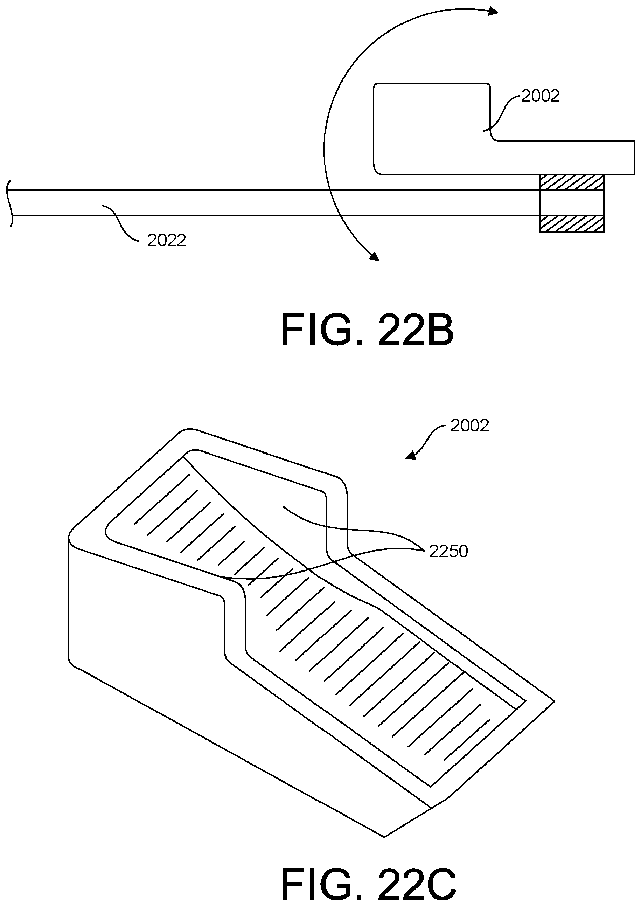

FIGS. 22A-22C show another variation of the exemplary gear assembly of FIGS. 20A-20F and/or 21A-21E. The actuator 2002 may be fixedly coupled to the cutter 2022. When the actuator 2002 is in the proximal position (FIG. 22A), it can be free to move distally. When the actuator is in the distal position (FIG. 22B), it can be free to rotate about the axis of the cutter 2022. After the user advances the actuator 2002 and cutter 2022 to the distal position, the user can rotate the actuator about 2002 the axis of the cutter 2022 causing the cutter 2022 to rotate (FIG. 22B). FIG. 22C shows interior walls 2250 of the actuator 2002. The interior walls 2250 may engage with the cutter 2022 to allow the user to rotate the cutter 2022 by pushing on the actuator 2002.

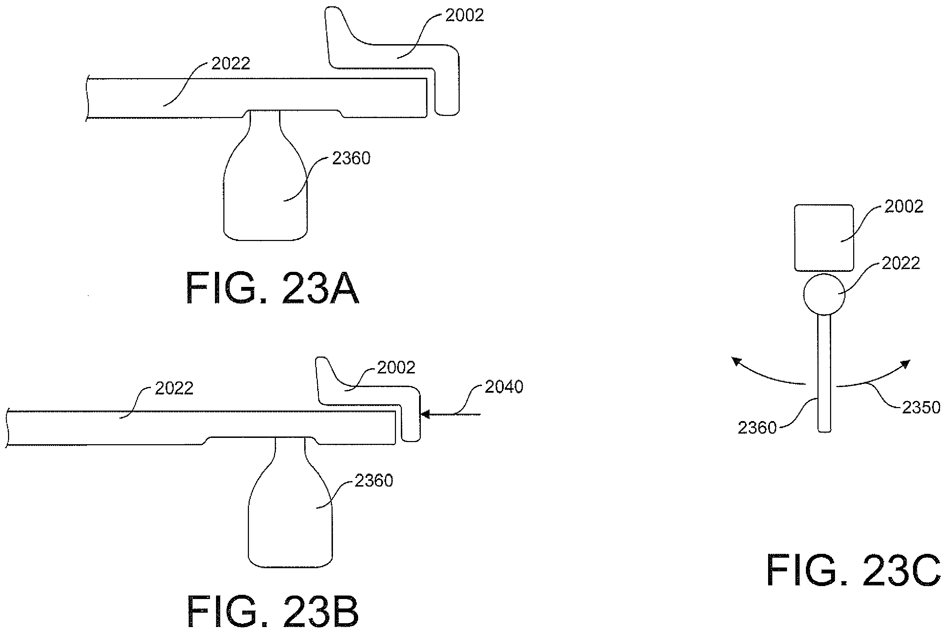

FIGS. 23A-23C show another variation of the exemplary gear assembly of FIGS. 20A-20F, 21A-21E and/or 22A-22C. A lever 2360 can be operationally coupled to the actuator 2002 and the cutter 2022. FIG. 23B shows that when the user advances the actuator 2002 distally 2340, cutter 2022 can advance distally with respect to the lever 2360. The front view of FIG. 23C shows that when the user toggles 2350 the lever 2360, this can cause the cutter 2022 to rotate. After the suture is cut, the user can release a catch to automatically allow the actuator 2002 and cutter 2022 to retract proximally, or the user can manually return the actuator 2002 to its proximal position.

When a feature or element herein is referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

In general, any of the apparatuses and methods described herein should be understood to be inclusive, but all or a sub-set of the components and/or steps may alternatively be exclusive, and may be expressed as "consisting of" or alternatively "consisting essentially of" the various components, steps, sub-components or sub-steps.

**As** used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/-0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative embodiments are described above, any of a number of changes may be made to various embodiments without departing from the scope of the invention as described by the claims. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes and should not be interpreted to limit the scope of the invention as it is set forth in the claims.

The examples and illustrations included herein show, by way of illustration and not of limitation, specific embodiments in which the subject matter may be practiced. As mentioned, other embodiments may be utilized and derived there from within the scope of the claims.

## Claims

1. A suture cutting device (200), comprising:
a handle (106);
an elongate holding tube (207) attached to the handle (106), the holding tube (207) including a slot (208) configured to allow a suture to pass into the holding tube (207);
an inner mandrel (209) within the elongate holding tube (207), the inner mandrel (209) configured to move relative to the elongate holding tube (207) to capture the suture in a channel (255) between the inner mandrel (209) and the elongate holding tube (207);
a tubular cutter (205, 1206, 1306, 1406) disposed around the elongate holding tube (207),
wherein the tubular cutter (205, 1206, 1306, 1406) is configured to move axially relative to the elongate holding tube (207),
**characterized in that** the tubular cutter (205, 1206, 1306, 1406) is configured to rotate relative to the elongate holding tube (207) such that a cutting edge (216) of the tubular cutter (205, 1206, 1306, 1406) cuts the suture when captured within the channel (255); and
the suture cutting device comprises an actuator (1315, 1415) configured to control rotation of the tubular cutter (205, 1206, 1306, 1406) for cutting the suture,
wherein the tubular cutter (205, 1206, 1306, 1406) is operationally coupled to a threaded collar (1209, 1309, 1409) that is configured to rotate and axially translate the tubular cutter (205, 1206, 1306, 1406).

2. The device of claim 2, wherein the tubular cutter (205, 1206) is configured to simultaneously rotate and move axially relative to the elongate holding tube (207).

3. The device of claim 2, wherein the tubular cutter (205, 1306, 1406) is configured to sequentially rotate and move axially relative to the elongate holding tube (207).

4. The device of claim 1, wherein the actuator (1315, 1415) is configured to be activated in at least two modes, wherein activating the actuator (1315, 1415) in a first mode causes the tubular cutter (1306, 1406) to rotate and activating the actuator (1315, 1415) in a second mode causes the tubular cutter (1306, 1406) to move axially.

5. The device of claim 1, wherein the actuator (1315, 1415) is configured to rotate the tubular cutter (1206, 1306, 1406) in a first direction when the actuator (1315, 1415) is activated and the tubular cutter (1206, 1306, 1406) rotates in an opposite direction when the actuator (1315, 1415) is released.

6. The device of claim 1, wherein the threaded collar (1409) comprises a flat external surface (1430) configured to slide along a flat surface of the handle (1450) such that actuation of the actuator (1415) axially slides the tubular cutter (1406) and prevents the threaded collar (1409) and tubular cutter (1406) from rotating while the threaded collar flat external surface (1430) engages the flat surface of the actuator (1415).

7. The device of claim 6, wherein the threaded collar flat external surface (1430) is configured to axially slide along the flat surface of the actuator (1415) and thereby axially slide the tubular cutter (1409), the actuator flat surface terminating such that further axial sliding of the threaded collar flat surface (1430) disengages the flat surfaces from each other and allows the threaded collar (1409) and tubular cutter (1406) to rotate.

## Patentansprüche

1. Nahtfadenschneidevorrichtung (200), umfassend:
einen Griff (106);
ein längliches Halterohr (207), das an dem Griff (106) angebracht ist, wobei das Halterohr (207) einen Schlitz (208) aufweist, der dazu ausgelegt ist, zu erlauben, dass ein Nahtfaden in das Halterohr (207) übergehen kann;
einen Innendorn (209) in dem länglichen Halterohr (207), wobei der Innendorn (209) dazu ausgelegt ist, sich bezogen auf das längliche Halterohr (207) zu bewegen, um den Nahtfaden in einem Kanal (255) zwischen dem Innendorn (209) und dem länglichen Halterohr (207) zu erfassen;
eine röhrenförmige Schneideinrichtung (205, 1206, 1306, 1406), die um das längliche Halterohr (207) angeordnet ist,
wobei die röhrenförmige Schneideinrichtung (205, 1206, 1306, 1406) dazu ausgelegt ist, sich axial bezogen auf das längliche Halterohr (207) zu bewegen,
**dadurch gekennzeichnet, dass** die röhrenförmige Schneideinrichtung (205, 1206, 1306, 1406) dazu ausgelegt ist, sich bezogen auf das längliche Halterohr (207) zu drehen, so dass eine Schneidkante (216) der röhrenförmigen Schneideinrichtung (205, 1206, 1306, 1406) den Nahtfaden schneidet, wenn er in dem Kanal (255) erfasst wird; und
die Nahtfadenschneidevorrichtung eine Betätigungseinrichtung (1315, 1415) umfasst, die dazu ausgelegt ist, die Drehung der röhrenförmigen Schneideinrichtung (205, 1206, 1306, 1406) zum Schneiden des Nahtfadens zu steuern,
wobei die röhrenförmige Schneideinrichtung (205, 1206, 1306, 1406) funktional mit einer Gewindemuffe (1209, 1309, 1409) gekoppelt ist, die dazu ausgelegt ist, die röhrenförmige Schneideinrichtung (205, 1206, 1306, 1406) zu drehen und axial zu translatieren.

2. Vorrichtung nach Anspruch 2, wobei die röhrenförmige Schneideinrichtung (205, 1206) dazu ausgelegt ist, sich gleichzeitig zu drehen und bezogen auf das längliche Halterohr (207) zu bewegen.

3. Vorrichtung nach Anspruch 2, wobei die röhrenförmige Schneideinrichtung (205, 1306, 1406) dazu ausgelegt ist, sich nacheinander zu drehen und bezogen auf das längliche Halterohr (207) axial zu bewegen.

4. Vorrichtung nach Anspruch 1, wobei die Betätigungseinrichtung (1315, 1415) dazu ausgelegt ist, in mindestens zwei Modi aktiviert zu werden, wobei das Aktivieren der Betätigungseinrichtung (1315, 1415) in einem ersten Modus bewirkt, dass sich die röhrenförmige Schneideinrichtung (1306, 1406) dreht, und das Aktivieren der Betätigungseinrichtung (1315, 1415) in einem zweiten Modus bewirkt, dass sich die röhrenförmige Schneideinrichtung (1306, 1406) axial bewegt.

5. Vorrichtung nach Anspruch 1, wobei die Betätigungseinrichtung (1315, 1415) dazu ausgelegt ist, die röhrenförmige Schneideinrichtung (1206, 1306, 1406) in eine erste Richtung zu drehen, wenn die Betätigungseinrichtung (1315, 1415) aktiviert wird, und sich die röhrenförmige Schneideinrichtung (1206, 1306, 1406) in eine entgegengesetzte Richtung dreht, wenn die Betätigungseinrichtung (1315, 1415) freigegeben wird.

6. Vorrichtung nach Anspruch 1, wobei die Gewindemuffe (1409) eine ebene Außenseite (1430) umfasst, die dazu ausgelegt ist, eine ebene Oberfläche des Griffs (1450) entlang zu gleiten, so dass die Betätigung der Betätigungseinrichtung (1415) die röhrenförmige Schneideinrichtung (1406) axial verschiebt und verhindert, dass sich die Gewindemuffe (1409) und die röhrenförmige Schneideinrichtung (1406) drehen, während die ebene Außenseite (1430) der Gewindemuffe die ebene Oberfläche der Betätigungseinrichtung (1415) in Eingriff nimmt.

7. Vorrichtung nach Anspruch 6, wobei die ebene Außenseite (1430) der Gewindemuffe dazu ausgelegt ist, die ebene Oberfläche der Betätigungseinrichtung (1415) entlang zu gleiten und dadurch die röhrenförmige Schneideinrichtung (1409) axial zu verschieben, wobei die ebene Oberfläche der Betätigungseinrichtung endet, so dass die weitere axiale Verschiebung der ebenen Oberfläche (1430) die ebenen Oberflächen voneinander löst und ermöglicht, dass sich die Gewindemuffe (1409) und die röhrenförmige Schneideinrichtung (1406) drehen.

## Revendications

1. Dispositif de découpe de suture (200), comprenant :
une poignée (106) ;
un tube de maintien allongé (207) fixé à la poignée (106), le tube de maintien (207) comportant une fente (208) conçue pour permettre le passage d'une suture dans le tube de maintien (207) ;
un mandrin interne (209) à l'intérieur du tube de maintien allongé (207), le mandrin interne (209) étant configuré pour se déplacer par rapport au tube de maintien allongé (207) afin de capturer la suture dans un canal (255) entre le mandrin interne (209) et le tube de maintien allongé (207) ;
un organe de coupe tubulaire (205, 1206, 1306, 1406) disposé autour du tube de maintien allongé (207),
dans lequel l'organe de coupe tubulaire (205, 1206, 1306, 1406) est configuré pour se déplacer axialement par rapport au tube de maintien allongé (207),
**caractérisé en ce que** l'organe de coupe tubulaire (205, 1206, 1306, 1406) est configuré pour tourner par rapport au tube de maintien allongé (207) de telle sorte qu'un tranchant (216) de l'organe de coupe tubulaire (205, 1206, 1306, 1406) coupe la suture lorsqu'elle est capturée à l'intérieur du canal (255) ; et
le dispositif de coupe de suture comprend un actionneur (1315, 1415) configuré pour commander la rotation de l'organe de coupe tubulaire (205, 1206, 1306, 1406) pour couper la suture,
dans lequel l'organe de coupe tubulaire (205, 1206, 1306, 1406) est accouplé de manière opérationnelle à une collerette filetée (1209, 1309, 1409) qui est configurée pour déplacer en rotation et en translation axiale l'organe de coupe tubulaire (205, 1206, 1306, 1406).

2. Dispositif selon la revendication 2, dans lequel l'organe de coupe tubulaire (205, 1206) est configuré pour tourner et simultanément se déplacer axialement par rapport au tube de maintien allongé (207).

3. Dispositif selon la revendication 2, dans lequel l'organe de coupe tubulaire (205, 1306, 1406) est configuré pour tourner et séquentiellement se déplacer axialement par rapport au tube de maintien allongé (207).

4. Dispositif selon la revendication 1, dans lequel l'actionneur (1315, 1415) est configuré pour être activé dans au moins deux modes, dans lequel l'activation de l'actionneur (1315, 1415) dans un premier mode provoque la rotation de l'organe de coupe tubulaire (1306, 1406) et l'activation de l'actionneur (1315, 1415) dans un second mode provoque le déplacement axial de l'organe de coupe tubulaire (1306, 1406).

5. Dispositif selon la revendication 1, dans lequel l'actionneur (1315, 1415) est configuré ; pour faire tourner l'organe de coupe tubulaire (1206, 1306, 1406) dans une première direction lorsque l'actionneur (1315, 1415) est activé et l'organe de coupe tubulaire (1206, 1306, 1406) tourne dans une direction opposée lorsque l'actionneur (1315, 1415) est relâché.

6. Dispositif selon la revendication 1, dans lequel le collier fileté (1409) comprend une surface externe plate (1430) configurée pour coulisser le long d'une surface plate de la poignée (1450) de telle sorte que l'actionnement de l'actionneur (1415) fasse coulisser axialement l'organe de coupe tubulaire (1406) et empêche le collier fileté (1409) et l'organe de coupe tubulaire (1406) de tourner pendant que la surface externe plate (1430) du collier fileté vient en prise avec la surface plate de l'actionneur (1415).

7. Dispositif selon la revendication 6, dans lequel la surface externe plate (1430) du collier fileté est configurée pour coulisser axialement le long de la surface plate de l'actionneur (1415) et de ce fait faire coulisser axialement l'organe de coupe tubulaire (1409), la surface plate de l'actionneur se terminant de telle sorte qu'un coulissement axial supplémentaire de la surface plate (1430) du collier fileté libère les surfaces plates l'une de l'autre et permette au collier fileté (1409) et à l'organe de coupe tubulaire (1406) de tourner.
